# EUROPEAN PATENT SPECIFICATION

(11) **EP 0 939 644 B1**
(45) Date of publication and mention of the grant of the patent: **14.01.2004**
(21) Application number: 97937379.2
(22) Date of filing: 27.08.1997
(51) Int. Cl.: A61K 38/42, A61K 31/555, A61K 38/18, A61K 35/14, A61K 35/28, A61K 45/06, C12N 5/06, C12N 5/08

(54) **ENHANCED STIMULATION OF ERYTHROPOIESIS**
GESTEIGERTE STIMULATION DER ERYTHROPOESIS
STIMULATION AMELIOREE DE L'ERYTHROPOIESE

(30) Priority: 27.08.1996 US 24632 P
(43) Date of publication of application: 08.09.1999
(73) Proprietor: Hemosol Inc., Mississauga Ontario L5N 8H9 (CA)
(72) Inventor: BELL, David, Oakville, Ontario L6H 4A7 (CA); MUELLER, Susan, G., Milton, Ontario L9T 2X7 (CA)
(74) Representative: Eyles, Christopher Thomas
(86) International application number: PCT/CA1997/000601
(87) International publication number: WO 1998/008537

(56) References cited:
- WO-A-92/02242
- WO-A-93/09220
- WO-A-95/24213
- J. HARRISON ET AL.: "ADDITIVE EFFECT OF ERYTHROPOIETIN AND HEME ON MURINE HEMATOPOIETIC RECOVERY AFTER AZIDOTHYMIDINE TREATMENT." BLOOD, vol. 82, no. 12, 15 December 1993, NEW YORK, N.Y., US, pages 3574-3579, XP002049731
- F. CORMIER ET AL.: "D VELOPPEMENT DES PROG NITEURS RYTHRO DES PR COCES (BFUe) DE LA MOELLE OSSEUSE DE SOURIS DANS UN MILIEU DE CULTURE D POURVU DE S RUM. EFFET DE L'H MINE." COMPTES RENDUS DES SEANCES DE L'ACADEMIE DES SCIENCES. SERIE III: SCIENCES DE LA VIE., vol. 299, no. 6, 30 July 1984, MONTREUIL FR, pages 143-146, XP002049732

## Description

### Field of the invention

The present invention relates the use of erytropoietin in combination with hemoglobin for the manufactore of a medicament for use in a novel method of stimulating erythropoiesis in mammals through the administration of a suitable amount of hemoglobin *in vivo* under conditions of decreased erythropoietin levels.

### Background of the Invention

### Stimulation of Erythroid Progenitors by Erythropoietin

Erythropoiesis is an essential process required to replace worn out red blood cells that are continuously removed from the circulation. Some 200 billion red blood cells, having an average life span of 120 days, are produced daily in adults. Under normal physiological conditions, erythropoiesis is principally regulated by erythropoietin (Epo), a hormone produced by the kidney in response to hypoxia. Erythropoietin, produced by the renal peritubular endothelium, circulates to the bone marrow where it stimulates committed stem cell progeny called erythroid progenitors to produce red blood cells (Krantz, Blood 77:419-34, 1991; Roberts and Smith, J. Mol. Endocrin. 12:131-48, 1994). Including platelets and white blood cells, the total daily blood cell production amounts to half a trillion cells. This level of cell replacement constitutes only the steady state condition and reflects the remarkable endogenous proliferative capacity of stem cells.

Two kinds of functionally distinct erythroid progenitors have been identified based on their abilities to form morphologically recognizable colonies when grown in semi-solid media such as methyl cellulose. The first, the burst forming unit-erythroid (BFU-E), represents the most primitive erythroid progenitor and forms large multi-clustered hemoglobinized colonies. The second, the colony forming unit-erythroid (CFU-E), is a more differentiated erythroid progenitor which forms smaller hemoglobinized colonies. The BFU-E is the earliest identifiable progenitor fully committed to erythropoiesis and has a larger capacity for self-renewal than the more mature CFU-E. Most BFU-E are quiescent with only 10 - 20% of the cells cycling at a given time, whereas, the majority of CFU-E are actively dividing. As BFU-E differentiate into CFU-E there is a loss in the expression of the primitive stem cell surface glycoprotein CD34, and an increase in the expression of receptors for erythropoietin and the iron transporter, transferrin. Although BFU-E express low numbers of receptors for erythropoietin, they are stimulated by Epo to proliferate and differentiate into CFU-E which, in turn, express higher levels of the Epo receptors. Erythroid differentiation beyond the CFU-E stage is dependent upon erythropoietin, and is characterized by the expression of the red blood cell membrane protein glycophorin A, the accumulation of additional erythroid-specific membrane proteins and the induction of hemoglobin synthesis.

### Role of Erythropoietin in Erythroid Cell Proliferation and Differentiation

Erythropoietin. stimulates erythroid proliferation and differentiation by interacting with a specific receptor expressed almost exclusively on erythroid progenitors. The Epo receptor is a member of the cytokine receptor superfamily and possesses the characteristic pentapeptide WSXWS motif (trp-ser-x-trp-ser, SEQ ID NO I ), along with four conserved cysteine residues within the extracellular domain (Krantz, Blood 77:419, 1991; Roberts and Smith, J. Mol. Endocrin. 12:131-48, 1994). Other members of the cytokine receptor superfamily include receptors for interleukin 2 (IL-2; β- and γ-chains), IL-3, IL-4, IL-6, IL-7, granulocyte-macrophage colony stimulating factor (GM-CSF), growth hormone and prolactin. All of these receptors have a similar predicted tertiary extracellular structure. The binding of erythropoietin to the Epo receptor results in the phosphorylation of the intracellular tyrosine kinase, JAK2, which, in turn, phosphorylates several intracellular proteins including STATS, PI3 kinase, *vav* and others (Ihle, Nature 377:591-94, 1995). Evidence suggests that activation of these second messengers, and others, by phosphorylation contributes to the Epo-induced proliferative response; however, the molecular basis which determines whether an erythroid cell will either proliferate or differentiate in response to Epo is unknown.

The later stages of erythroid differentiation are best characterized by the accumulation of the major red blood cell protein, hemoglobin, a tetrameric molecule consisting of an oxygen-binding heme moiety bound to each of four separate globin chains. At a concentration of ~28 pg/cell, hemoglobin is the most abundant protein present in the mature red blood cell, accounting for 95% of the cell protein. The high rate of red blood cell production in the marrow requires that red blood cell precursors synthesize 400 trillion molecules of hemoglobin every second. Erythropoietin-stimulated hemoglobin synthesis is coordinated within differentiating red cell precursors so that the synthesis of the constituent alpha and beta globin chains is concurrent with that of heme. Globin genes and genes encoding multiple enzymes along the heme-synthesis pathway (Weiss and Orkin, Exper. Hematol. 23:99-107, 1995) are transactivated by the major erythroid transcription factor, GATA-1, which is expressed following the activation of the Epo receptor by the binding of Epo (Chiba et al., Nuc. Acid Res. 19:3843-48, 1991; Dalyot et al., Nuc. Acid Res. 21:4031-37, 1993; Busfield et al., Eur. J. Biochem. 230:475-80, 1995). Whether Epo will support primarily erythroid differentiation or proliferation appears to depend on the concentration of Epo and the status of the cell cycle. Low concentrations of Epo support β-globin production and prolong the G1 phase of the cell cycle, whereas higher Epo concentrations promote cell proliferation and shorten the G1 phase (Carroll et al., Proc. Natl. Acad. Sci. USA 92:2869-73, 1995).

### Current Treatments of Anemia

Anemia is the pathological consequence of insufficient hemoglobin levels to meet the oxygen transport requirements of the body. There are several causes of anemia which include excessive blood loss, increased red blood cell destruction, decreased red blood cell production or hemoglobin synthesis, and abnormal hemoglobin production. Decreased red blood cell production may result from inadequate iron incorporation (either iron deficiency or failure of iron mobilization, as seen in anemia of chronic disease), insufficient Epo production or bone marrow failure. Since the erythropoietic activity of bone marrow is intact in iron and Epo-dependent anemias, such anemias are treatable by iron or Epo administration, respectively.

A variety of different disorders result in Epo-insufficiency, but the most classic examples are the diseases of the kidney. Patients with chronic renal failure typically exhibit Epo-dependent anemia due to the inability of their damaged kidneys to produce Epo. These patients require frequent dialysis to replace kidney function, and 90% of patients are clinically anemic. Until recently, the treatment of anemia in dialysis patients was via multiple transfusions. With the advent of recombinant human Epo, transfusions have been largely replaced by the administration of Epo. Indeed, ~88% of all dialysis patients are treated with Epo. Dialysis patients typically respond to Epo therapy with increases in reticulocyte count, hemoglobin level and hematocrit (Dunn and Markham, Drugs 51:299-318, 1996). Epo therapy is not without its drawbacks. One third of patients develop hypertension, which can generally be corrected using anti-hypertensive drugs. Iron deficiencies can also develop due to the increased transfer of iron from stores within the bone marrow to the rapidly proliferating erythroid progenitors for use in hemoglobin synthesis. The effectiveness of Epo therapy is reduced by insufficient iron and, thus, iron must generally be administered in conjunction with Epo for long-term therapy.

Erythropoietin is also approved for the treatment of anemia caused by chronic renal insufficiency, cancer or cancer therapy, as well as in patients infected with human immunodeficiency virus (HIV) who are undergoing zidovudine therapy. As the clinical applications of recombinant human erythropoietin expand, the cost of long-term therapy becomes a major concern. Typical Epo doses for dialysis patients are 225 Units/kg/week, administered in three doses. Medicare reimbursement for Epo treatment in the U.S. is presently $10.00 per 1,000 Units (Section 13566, Omnibus Budget Reconciliation Act of 1993). Thus, the typical cost for a 70 kg patient would be ~$8,000 yearly. In 1995, 175,000 patients were on dialysis in the U.S. resulting in a market excess of $883 million for this indication alone (Amgen, 1995; Annual Report). The cost of this therapy is projected to be ~$1.1 billion in 1996 (Dau Hoffman; 1996, Vector Securities International). Novel therapies which would reduce the requirement for Epo in the treatment of anemia would thus be of benefit to patients and the health care system. A recent study suggests that Epo requirements could be substantially (~46%) reduced through the administration of intravenous iron (Fishbane et al., Am. J. Kid. Dis. 26:41-46, 1995). However, tissue iron overload is of concern. The discovery of other agents capable of reducing Epo requirements for the treatment of Epo-dependent anemias without producing iron overload would be advantageous.

### Early Studies with Hemoglobin

As early as the 1940s, an increase in both blood hemoglobin and hematocrit was observed in anemic patients which received multiple injections of unmodified human hemoglobin (Amberson et al., J. Appl. Physiol. 1:469-89, 1994). The increases observed in these patients could have been due to the hemoglobin itself or to the efficient delivery of iron contained therein. However, the high levels of dissociated αβ globin dimers in the initial crude hemoglobin preparations resulted in renal tubular obstruction and decreased glomerular filtration rates (Spence, *Clinical Practice of Transfusion Medicine,* 3rd Edition, chapter 44, 1996). These crude hemoglobin preparations were also contaminated with endotoxin, residual cellular stroma and lipid fragments, all of which could contribute to adverse side effects including vasoconstriction, complement activation and the generation of free radicals. As a result of their adverse side effects which included hypertension, shock, renal damage, anaphylaxis and sometimes death, early studies using crude hemoglobin preparations were discontinued. A direct role for hemoglobin in the induction of erythropoiesis was never established.

### Crosslinked Hemoglobin

More recently, relatively nontoxic highly purified crosslinked hemoglobins have been developed primarily for use as blood substitutes. The crosslinked hemoglobins are stabilized to prevent the dissociation of hemoglobin into αβ dimers and, thus, do not adversely affect the kidneys. Furthermore, the steps used in purifying hemoglobin for crosslinking have reduced the adverse side effects previously observed with the earlier crude hemoglobin preparations. In a recent study, the infusion of a blood substitute, crosslinked bovine hemoglobin, into patients suffering from aplastic anemia was observed to significantly increase the number of circulating reticulocytes in three days, and the level of blood hemoglobin in seven days (Feola et al., Surg. Gynecol. Obstet. 174:379-86, 1992). However, no assessment of the effect of hemoglobin on hematopoiesis could be determined. The concurrent use of antimalarial and antibiotic drugs clouded the study's results. Although the authors stated that there was an erythropoietic effect, no direct studies were performed. Accordingly, the conclusions drawn by the study's authors must be interpreted as generalization. In fact, an independent assessment of this study attributed the clinical observations to the increased iron provided by the hemoglobin and not to the stimulation of erythropoiesis (Spence, *Clinical Practice of Transfusion Medicine,* chapter 44, 1996).

In another recent study, the hematological effects of crosslinked bovine hemoglobin were measured on human subjects (Hughes et al., J. Lab Clin. Med. 126:444-51, 1995). All subjects underwent a partial phlebotomy to remove~15% of their blood volume, followed by a 3:1 hemodilution with Ringer's lactate and an intravenous injection of up to 45 g of crosslinked hemoglobin or the control solution (Ringers lactate). Serum iron levels peaked at 8 hours and paralleled changes in the plasma levels of crosslinked hemoglobin, whereas ferritin levels peaked at 48 hours post-infusion. These data are consistent with the release of iron from the hemoglobin after it had been metabolized by the reticuloendothelial system. Serum Epo levels increased two- to six-fold after 24 hours in all groups, but to a greater extent in subjects that received hemoglobin. The elevated Epo levels were considered an indirect effect attributed to hypoxemia induced by the phlebotomy/hemodilution procedure itself. No significant difference was observed in the hemoglobin or reticulocyte levels in the control or hemoglobin treated groups. The observed increase in Epo levels was apparently insufficient to stimulate erythropoiesis. In a more recent report, 0.4 or 0.6 g/kg body weight of the same crosslinked bovine hemoglobin was administered post-operatively to patients undergoing elective surgery (Hughes et al., Crit. Care Med. 24:756-64, 1996). Both the hematocrit and the corrected absolute reticulocyte count increased in patients treated with hemoglobin. Again, the reported stimulation of erythropoiesis by hemoglobin could not be distinguished from the effects of hemoglobin-mediated iron delivery, or the stimulation of erythropoiesis through an alternate mechanism.

*In vitro* studies demonstrated that crosslinked hemoglobin may protect erythroid progenitors from the toxic effects of 3'-azido-3'-deoxythymidine (AZT) (Fowler et al., Toxicol. Letts. 85:55-62, 1996). AZT can significantly inhibit the proliferation of erythroid cells in cultures of human CD34⁺ bone marrow cells. Low doses of crosslinked recombinant human hemoglobin (0.01 - 1 *µ*M) did not increase the proliferation of the erythroid cultures; however, when combined with AZT, the crosslinked hemoglobin reversed its toxic effects. A direct interaction of AZT with hemoglobin can not be ruled out as contributing to the reduced toxicity of the AZT in this study. Thus, *in vivo* data which has suggested a role for hemoglobin in the stimulation of erythropoiesis has yet to be directly demonstrated *in vitro.*

### Stimulation of Erythroid Cells by Heme

Hemin, the ferric chloride salt of heme, has been shown to promote erythroid progenitor proliferation and differentiation in a variety of *in vitro* assays. Heme is the end product of a tightly regulated multi-enzymatic pathway, part of which occurs within the mitochondria. Intracellularly, heme is the prosthetic group for hemoproteins which include hemoglobin, catalase and the cytochromes. Heme is involved in the regulation of the intracellular synthesis of these proteins at various levels including gene transcription, mRNA translation, transport, assembly and/or protein turnover (Padmanaban et al., Trends Biochem. Sci. 14:492-96, 1992). Exogenously added hemin induces erythroid differentiation in a number of erythroleukemic cell lines resulting in hemoglobin production (Ross and Sautner, Cell 8:513-20, 1976; Rutherford et al., Nature 280:164-65, 1979; Dean et al., Science 212:459-61, 1981). The stimulation of hemoglobin production by hemin is due to increases in both globin gene transcription and globin mRNA stability (Ross and Sautner, Cell 8:513-20, 1976). Hemin treatment specifically increases embryonic and fetal globin production in human cell lines and primary human erythroid cells without affecting β-globin production (Rutherford et al., Nature 280:164-65, 1979; Fibach et al., Blood 85:2967-74, 1995). Although hemin alone can induce erythroid differentiation of erythroleukemic cell lines, hemin requires the addition of exogenous Epo to stimulate differentiation of primary cultures of erythroid cells (Fibach et al., Blood 85:2967-74, 1995).

Along with its effect on erythroid differentiation, hemin also exerts a proliferative effect on erythroid progenitors. The *in vivo* administration of hemin into mice results in increases in BFU-E within the marrow (Monette et al., Exp. Hematol. 12:782-87, 1984). BFU-E colonies that formed in response to hemin treatment were larger and appeared earlier in culture than those from untreated samples (Holden et al., Exp. Hematol. 11:953-60, 1983). *In vitro,* hemin (50 - 200 *µ*M) stimulates a two-fold increase in murine erythroid colonies over those stimulated by 0.1 U/ml Epo alone (Porter et al., Exp. Hemat. 7:11-16, 1979). In this study, hemin also stimulated erythroid colony formation in the absence of added Epo. Thus, hemin, which can be directly incorporated into hemoglobin by erythroid cells, may also influence both the proliferation and differentiation of erythroid cells.

The effects of hemin could be explained by the delivery of only iron; however, equimolar concentrations of the iron salt ferric chloride are unable to stimulate erythroid progenitor proliferation and/or differentiation to the same extent as hemin. Thus, it is unlikely that hemin stimulates erythroid cells simply through the delivery of iron. *In vivo* the likely source of hemin (or heme) is from the breakdown of hemoglobin. One potential mechanism for the erythropoietic activity of hemoglobin is through the delivery of heme following its dissociation from its globin carriers. Free heme could be released from hemoglobin prior to its uptake by erythroid progenitors or, alternatively, intact hemoglobin could be taken up by the cells prior to the release of heme intracellularly. Intracellular heme could then stimulate erythroid progenitor proliferation and differentiation as previously described for hemin. Whether such a pathway occurs *in vivo* is presently unclear from the literature. Generally hemoglobin, which is released from lysed red blood cells, is cleared efficiently from the circulation. Free hemoglobin is bound in the circulation by haptoglobin and this complex is transported to the liver where it is rapidly cleared by hepatocytes. If haptoglobin becomes saturated then unbound hemoglobin is oxidized leading to the dissociation of heme from the globin chains. Free heme is then bound by hemopexin and transported to the liver where the heme group is either degraded to bilimbin or incorporated into cytochrome P-450 (Otto et al., Crit. Rev. Microbiol. 18:217-33, 1992). In studies investigating the role of hemin in erythroid progenitor proliferation and differentiation, there are no indications or suggestions of the potential role of hemoglobin in erythropoiesis, either directly or through the provision of heme.

### Summary of the Invention

The present invention overcomes the problems and disadvantages associated with current strategies and designs and the present specification describes new compositions and methods for the treatment of human disorders.

The invention is directed to the use of erythropoietin in combination with hemoglobin for the manufacture of a medicament for enhancing erythropoiesis in a patient, wherein the amount of erythropoietin to be administered is from 22.5 to 42.9 U/kg/week and the amount of hemoglobin to be administered is 85 mg/kg/week.

Other embodiments and advantages of the invention are set forth, in part, in the description which follows and, in part, will be obvious from this description or may be learned from the practice of the invention.

### Description of the Drawings

- Figure 1: Fold expansion of umbilical cord blood progenitors at ambient oxygen.
- Figure 2: Fold expansion of umbilical cord blood progenitors at 5% oxygen.
- Figure 3: Fold expansion of umbilical cord blood progenitors with 0.2 Units of Epo at ambient oxygen.
- Figure 4: Fold expansion of umbilical cord blood progenitors with 0.2 Units of Epo at 5% oxygen.
- Figure 5: Representative erythroid colony formation with various additions.
- Figure 6: Representative HPLC profiles of the analysis of hemoglobin production.
- Figure 7: Representative 3-dimensional frequency distributions of erythroid progenitors induced to differentiate in liquid culture in (a) the absence or (b) the presence of HAo.
- Figure 8: Fold expansion of adult blood progenitors at ambient oxygen.
- Figure 9: Fold expansion of adult blood progenitors at 5% oxygen.
- Figure 10: Fold expansion of adult blood progenitors with 0.2 Units of Epo at ambient oxygen.
- Figure 11: Fold expansion of adult blood progenitors with 0.2 Units of Epo at 5% oxygen.
- Figure 12: Fold expansion of CD34* progenitors with 2 Units of Epo at 5% oxygen.

### Description of the Invention

As embodied and broadly described herein, the present invention is directed to the use of erythropoietin in combination with hemoglobin for the manufacture of a medicament for enhancing erythropoiesis in a patient, wherein the amount of erythropoietin to be administered is from 22.5 to 42.9 U/kg/week and the amount of hemoglobin to be administered is 85 mg/kg/week.

### Role of Hemoglobin in the Treatment of Anemia

According to the current literature, iron alone is typically sufficient to augment the Epo-dependent stimulation of erythropoiesis. This can be simply attributed to the obligate requirement for iron in the synthesis of hemoglobin in response to Epo-stimulation. Hemin appears to be better than iron at enhancing Epo-stimulated erythropoiesis, but it may act independently of both iron, which it also provides in the form of heme, and of Epo through the direct activation of genes involved in hemoglobin synthesis. Hemin is more efficiently taken up and utilized by erythroid progenitors than free iron which can only be delivered to the progenitors via the receptor-mediated endocytosis of the specific plasma iron transporter, transferrin. Following the reduction of hemin by a ferri-reductase, the resultant heme can be directly incorporated into nascent globin chains during hemoglobin assembly while free iron requires endogenous porphyrin synthesis before it could be incorporated into heme, and subsequently hemoglobin. Hemoglobin is a source of both heme and iron, but also enhances Epo-dependent erythropoiesis through mechanisms that are distinct from both Epo and iron or heme delivery.

It has been surprisingly discovered that hemoglobin can be used to directly and specifically stimulate primitive erythroid progenitors in the presence of low doses of Epo at which erythroid progenitor growth is otherwise severely limited. The exact mechanism of hemoglobin stimulation is substantially different from that of stimulation by hemin. Thus, not only does hemoglobin directly stimulate erythroid cells, hemoglobin also provides a readily available source of heme with all of its erythroid-specific stimulatory activity and which may be used in the synthesis of hemoglobin. In this regard, *in vivo* hemoglobin can act synergistically with Epo in the stimulation of erythropoiesis and consequently lower the amount of Epo required to generate a clinically beneficial erythropoietic response in anemic patients. hemoglobin is matched by productive erythropoiesis *in vivo.* Hemoglobin is similar to hemin in the stimulation of erythroid proliferation, but is more effective than hemin in stimulating erythroid differentiation and results in significantly higher adult and fetal hemoglobin production. Thus, hemoglobin provides a stronger and more potent stimulus to developing erythroid cells than does heme which may be mediated by an independent mode of action. Nonetheless, part of hemoglobin's stimulatory activity resides in the effective delivery of heme which, in turn, stimulates erythroid cell proliferation and globin synthesis and which may be incorporated into hemoglobin. Thus, hemoglobin may be used to treat anemias due to reduced erythropoietin levels, and to lower the amount of erythropoietin administered to treat such anemias,

Hemoglobin as used herein includes, for example, natural or purified hemoglobin, recombinant hemoglobin, cross-linked hemoglobin such as, for example, those described in U.S. Patent Nos. 5,439,591; 5,545,328; and 5,532,352 (*e.g.* HEMOLINK™), hemoglobin fragments and chemically or genetically modified hemoglobin that, for example, prevent dissociation of the hemoglobin molecule or modify the oxygen-binding affinity, hemoglobin precursors, and hemoglobin in any oxidative state including the oxi and deoxy and met forms, nitric oxide (NO) and carbon monoxide (CO) forms and iron III (ferric) hemoglobin. Many of these forms of hemoglobin are commercially available such as, for example, HEMOLINK™ (an O-raffinose cross-linked hemoglobin). These and additional forms of hemoglobin are disclosed in U.S. Patent Nos. 4,857,636; 5,189,146; 5,364,932; 5,399,671; 5,532,352; 5,250,665; and 5,334,707; as are methods for the crosslinking of hemoglobins.

Preferably, compositions of the invention are pharmaceutical compositions that may contain one or more pharmaceutically acceptable carriers. Suitable pharmaceutically acceptable carriers include, for example, salts and salt solutions (e.g Ringer's lactate), alcohols, water, glycerol, glycol such as polyethylene glycol, vitamins, minerals, proteins such as albumin, glycerin, oils, fatty acids, salts such as sodium, saccharides and polysaccharides, amino acids, starches, and combinations of these carriers. Pharmaceutical compositions can be administered directly to the patient or stored concentrated for dilution before use. Ready-to-use and concentrated forms may contain stabilizers and preservatives such as anti-oxidants and buffers that increase the stability of the heme-containing component and the composition.

Compositions of the invention are preferably physiologically stable and safe at therapeutically effective concentrations. Physiological stable compositions contain heme-containing components that do not break down or otherwise become ineffective upon introduction to a patient prior to having a desired effect. Components can also be made structurally resistant to catabolism, and thus, physiologically stable, by electrostatic or covalent coupling to specific reagents to increase physiological stability. Such reagents include salts and salt solutions (*e.g.* Ringer's lactate), amino acids such as arginine, glycine, alanine, asparagine, glutamine, histidine or lysine, nucleic acids including nucleosides or nucleotides, or substituents such as carbohydrates, saccharides and polysaccharides, lipids, fatty acids, proteins, or protein fragments. Useful coupling partners include, for example, glycol such as polyethylene glycol, glucose, glycerol, glycerin and other related substances.

Compositions of the invention should also be safe at effective dosages. Safe compositions are compositions that are not substantially toxic, myelotoxic, mutagenic or teratogenic at required dosages, do not cause adverse reactions or side effects, and are well tolerated. Although side effects may occur, safe compositions are those wherein the benefits achieved from their use outweigh disadvantages attributable to adverse side effects. Unwanted side effects include nausea, vomiting, hepatic or renal damage or failure, hypersensitivity, allergic reactions, cardiovascular problems, gastrointestinal disturbances, seizures and other central nervous system difficulties, fever, bleeding or hemorrhaging, coagulation or thrombosis, serum abnormalities and respiratory difficulties.

Hemoglobin is typically obtained in large quantities from the blood of a mammal. Suitable mammals include primates, and preferably humans, and may also cattle, horses, sheep and swine. Blood may be obtained from specific areas or tissues such as peripheral blood obtained from an adult, child or infant (which can be used directly or proliferated *in vitro),* umbilical cord blood, blpod obtained from bone marrow, discarded blood from blood banks and slaughter houses. Recombinant techniques have also successfully yielded expression of large quantities of hemoglobin from prokaryotic and eukaryotic cells. Eukaryotic cells which can express hemoglobin include animal or plant cells, that have been genetically engineered or selected to express large amounts of hemoglobin protein. Large scale isolation and purification of hemoglobin can be performed by those of ordinary skill in the art using well-known and established procedures (*e.g.* U.S. Patent.No. 5,439,591 and 5,545,328).

Many of the experiments disclosed herein were conducted using adult human hemoglobin obtained from red blood cells and purified by displacement chromatography processes as described in U.S. Patents 5,439,591 and 5,545,328. This purified, but uncross linked product is referred to as HAo. Cross-linked hemoglobin used included the O-raffinose modified form of hemoglobin referred to as HEMOLINK™. While administration can be with either uncross linked human hemoglobin or stabilized crosslinked human hemoglobin, crosslinked, stabilized hemoglobin is preferred for *in vivo* administration to humans. The ability of cross-linked hemoglobin to stimulate erythropoiesis provides potential therapeutic applications for the use of hemoglobin in the treatment of anemia and other disorders. In most situations, unmodified hemoglobin cannot be used in humans as the tetrameric molecule readily dissociates into its substituent αβ dimers. These dimers are rapidly cleared from the bloodstream by the kidney which can result in renal damage. In contrast, hemoglobin can be modified to prevent tetramer dissociation via chemical or genetic approaches. Hemoglobins modified to prevent tetramer dissociation are not readily cleared by the kidney and consequently do not cause renal damage. HEMOLINK™, an example of a chemically crosslinked hemoglobin, is prepared by treating human hemoglobin with a polyaldehyde (O-raffinose) obtained by oxidatively ring-opening the saccharide raffinose, which crosslinks the hemoglobin tetramer across the 2,3-diphosphoglycerate binding site. Other crosslinking agents may be used to stabilize the hemoglobin tetramer, and such agents are well known in the art and described in the patent literature. HEMOLINK™ is discussed and used by way of example only.

### Stimulation of Erythropoiesis

The medicaments to which the invention relates can be used in methods for the stimulation of erythropoiesis. Methods comprise the administration of a therapeutically-effective amount of a hemoglobin -containing composition of the invention. An effective or a therapeutically effective amount is that amount of the composition or the hemoglobin-containing component of the composition that is effective at detectably stimulating erythropoiesis of cells, preferably erythroid cells, or cells of a patient. Erythropoiesis is detectable if, for example, stimulation can be observed in culture or stimulation overcomes one or more patient symptoms associated with lack of erythropoiesis. A therapeutical effective amount is relation to a disorder is that amount which has a beneficial effect to the patient by alleviating one or more symptoms of the disorder or simply reducing premature mortality. For example, a beneficial effect may be a decrease in pain, a decrease in duration, frequency or intensity of a symptom, an increased hemocrit, an improved erythropoiesis, an increased reticulocyte count, an increased red cell count, an increased total hemoglobin, an increased peripheral blood flow, a decreased hemolysis, decreased fatigue or an increased strength. Preferably, a therapeutic amount is that amount of the hemoglobin-containing component that stimulates or enhances the expression of hemoglobin such as, for example, adult or fetal hemoglobin globin, or the proliferation of fetal or adult hemoglobin expressing cells.

Patients that can be effectively treated by the compositions of the invention include patients that have a lower than normal level of Epo or less than normal ability to produce or express Epo. Surprisingly, hemoglobin-containing compositions work effectively in the presence of reduced amounts of Epo, unlike other treatments which require that high or at least normal levels of Epo be maintained in the patient. Reduced Epo concentration are concentrations that are less than the concentration found in otherwise normal patients or the average of otherwise normal patients. That amount of endogenous Epo in normal human adults is typically between about 10 to 20 mUnits of Epo per ml of peripheral blood. Accordingly, less than normal amounts are amounts that are less than about 10 mU/ml, such as less than about 5 mU/ml, preferably less than about 2 mU/ml, and more preferably less than about 1 mU/ml, or none.

In the treatment of many disorders including anemias such as ACD, Epo is administered to the patient in fairly large amounts. Target serum or blood plasma concentrations are desired to be greater than 1 U/ml, preferably greater than 2 U/ml, and more preferably greater than about 5 U/ml. Epo concentrations desired upon administration of a heme-containing component are less than about 5 U/ml, preferably less than about 2 U/ml, and more preferably less than about 1 U/ml, and even more preferably less than about 0.2 U/ml.

The invention envisages the co-administration of both a hemoglobin containing component and Epo. However, as the hemoglobin-containing component acts synergistically with Epo, the amount of Epo required for the same result is substantially reduced. This results in a substantial savings in time, cost and overall success of a treatment. For example, Epo treatments that can be performed using less Epo will reduce undesirable side effects that may be associated with Epo administration, can render the Epo that. is administered more effective to the patient, and reduces or eliminates problems associated with tolerance to the composition, all without decreasing the rate of success. In addition, as doses can be reduced, overall care may include other treatments in combination with Epo therapy.

Compositions of the invention are preferably specific for the stimulation of erythropoiesis in erythroid progenitors such as CFU-GEMM cells, BFU-E cells and CFU-E cells. Compositions preferably do not significantly stimulate the proliferation or differentiation of non-erythroid cells such as CFU-Baso cells, CFU-Mast cells, CFU-GM cells, CFU-Eo (Eosinophil) cells, and lymphoid progenitor cells (CFU-L). A stimulation is not significant if the resulting effect is not either beneficial or harmful to the results of the treatment. The stimulation of erythropoiesis may include the stimulation of differentiation of erythroid cells, or the increased expression of hemoglobin such as adult or fetal hemoglobin. Surprisingly, stimulation can involve the stimulation of expression of both adult and fetal forms of hemoglobin.

The invention is also useful for the treatment of anemic patients to ameliorate one or more symptoms associated with the disorder. As the hemoglobin containing composition acts synergistically with Epo, combination treatments of hemoglobin plus Epo can be considered and will be more successful that either single treatment alone. Further, in many anemic disorders, patients have a fairly low endogenous level of Epo which may be the cause, effect or simply a consequence of the anemia.

Disorders that can be treated include diseases and maladies that can be characterized as a direct or indirect consequence of a defect of hematopoiesis, a defect in the production or expression of hemoglobin, or a defect or deficiency in erythroid cell differentiation and development. Such disorders include, for example, anemias such as sickle cell anemia, hemolytic anemia, infectious anemia, aplastic anemias, hypoproliferative or hypoplastic anemias, sideroblastic anemias, myelophthisic anemias, antibody-mediated anemias, anemias due to enzyme-deficiencies or chronic diseases, anemias due to blood loss, radiation therapy or chemotherapy, thalassemia including α-like and β-like thalassemia, or globin disorders due to infections of viral, bacterial or parasitic origin such as malaria, trypanosomiasis, human immunodeficiency virus and other retroviruses, a polyoma virus such as JC virus, a parvovirus, or a hepatitis virus such as human hepatitis viruses types A-G. Treatable disorders also include syndromes such as hemoglobin C, D and E disease, hemoglobin lepore disease, and HbH and HbS diseases.

Additional disorders that can be treated by the compositions of the invention include disorders associated with iron and heme deficiencies including porphyria such as acute hepatic porphyria. Hemoglobin containing components of the composition provide excellent vehicles for transferring iron or heme, in an acceptable form, into cells for the production of hemoglobin, cellular proteins and enzymes, such as all cytochromes including cytochrome p450, and other functions associated with cellular iron or heme. The administration of useable iron is also important in the treatment of disorders such as anemia of chronic disease (ACD), and in bone marrow transplants. Premature newborns often require stimulation of erythropoiesis with the concomitant addition of iron. However, newborn typically have very low levels of Epo and do not respond well to the direct administration of iron or iron infusion complexes such as INFUFER^{TM}. These patients can especially benefit with the administration of compositions of the invention.

Treatment with compositions of the invention ameliorates one or more symptoms associated with a disorder. Symptoms typically associated with disorders associated with erythropoiesis include, for example, anemia, tissue hypoxia, organ dysfunction, porphyria, abnormal hematocrit values, ineffective erythropoiesis, abnormal reticulocyte (erythrocyte) count, abnormal iron load, the presence of ring sideroblasts, splenomegaly, hepatomegaly, impaired peripheral blood flow, dyspnea, increased hemolysis, jaundice, anemic crises and pain such as angina pectoris.

The patient may be a domesticated animal such as a dog. cat, horse, cow, steer, pig, sheep, goat or chicken, or a wild animal, but is preferably a primate such as a human. Administration may be to an adult, an adolescent, a child, a neonate, an infant or *in utero.*

The treatment of newborns with compositions of the invention is beneficial as newborns and other neonates typically have very low levels of Epo. Accordingly, treatment of newborns and newborns who may be suffering from hemoglobin deficiency would substantially benefit from the compositions of the invention.

Administration of the composition may be short term, continuous or sporadic as necessary. Patients with a suspected or diagnosed with a erythropoietic disorder may only require composition treatment for short periods of time or until symptoms have abated or have been effectively eliminated.

Preferably, patients that can benefit from the methods of the invention are patients undergoing cell transplantation such as, for example, stem cell transplantation by bone marrow replacement, cord blood transplantation, leukophoresis, mobilized adult peripheral blood. In, for example, mobilized adult peripheral blood transplantation, peripheral blood is obtained from the patient and treated with one or more cytokines to promote differentiation of proliferation of cells such as stem cells or progenitor cells. Treated cells are than mobilized or infused into the same or an immunogenically matched patient after the patient was subjected to radiotherapy or chemotherapy. In this manner, transplanted cells, which may be an expanded population of the patient's own cells, can re-populate the otherwise cell depleted patient. Red blood cell engraftment can be enhanced as well as bone marrow replacement and cell enrichment following leukophoresis.

Patients undergoing chemotherapy or radiation therapy can also benefit from the compositions of the invention. Surprisingly, it has been discovered that compositions of the invention can reduce toxicity associated with other forms of therapy. For example, cancer patients being treated or about to be treated with chemotherapy can be co-administered a hemoglobin containing composition of the invention. Cellular toxicity of the chemotherapeutic agent is substantially reduced by the hemoglobin-containing component. Substantially reduced means that toxicity is reduced such that treatment may continue, that side effects attributed to the treatment can be more easily tolerated by the patient, or that increased amounts of the chemotherapeutic agent can be utilized. Chemotherapeutic agents which show this affect include nucleoside analogs such as, for example, acyclovir (ACV), ganciclovir (GCV), famciclovir, foscarnet, ribavirin, zalcitabine (ddC), zidovudine or azidothymidine (AZT), stavudine (D4T), larnivudine (3TC), didanosine (ddI), cytarabine, dideoxyadenosine, edoxudine, floxuridine, idozuridine, inosine pranobex, 2'-deoxy-5-(methylamino)uridine, trifluridine and vidarabine. Examples of protease inhibitors that show this affect include saquinivir, ritonavir and indinavir. Other agents include the cyclophosphamide such as alkylating agents, the purine and pyrimidine analogs such as mercaptopurine, the vinca and vinca-like alkaloids, the etoposides or etoposide like drugs, the antibiotics such as deoxyrubocin and bleomycin, the corticosteroids, the mutagens such as the nitrosoureas, antimetabolites including methotrexate, the platinum based cytotoxic drugs, the hormonal antagonists such as antiinsulin and antiandrogen, the antiestrogens such as tamoxifen an other agents such as doxorubicin, L-asparaginase, DTIC, mAMSA, procarbazine, hexamethylmelamine and mitoxantrone.

Compositions can be directly or indirectly administered to the patient. Indirect administration is performed, for example, by administering the composition to cells *ex vivo* and subsequently introducing the treated cells to the patient. The cells may be obtained from the patient to be treated or from an immunologically matched or unmatched patient, or a genetically related or unrelated patient (*e.g.* syngeneic or allogeneic cells). Related patients offer some advantage by lowering the immunogenic response to the cells to be introduced. For example, using techniques of antigen matching, immunologically compatible donors can be identified and utilized. Administration for *in vivo* stimulation can be by any means that is safe and effective for the patient.

Direct administration of a composition may be by parenteral administration, or by pulmonary absorption such as sprays to nasal areas which can provide rapid access to the bloodstream. Parenteral administration may be by intravenous injection, intra-arterial injection or direct injection or other administration to one or more specific sites. Injectable forms of administration are sometimes preferred for maximal effect in, for example, bone marrow. Administration can be by bolus injection or sequential over time (episodically) such as every one, two, four, six or eight hours, or every day (QD), or every other day (QOD). When long term administration by injection is necessary, venous access devices such as medi-ports, in-dwelling catheters, or automatic pumping mechanisms are also preferred wherein direct and immediate access is provided to the arteries in and around the heart and other major organs and organ systems. Effective *in vitro* amounts are typically less than therapeutically effective *in vivo* amounts as *in vivo,* the component distributes throughout the body. However, concentrations in specific areas such as, for example, bone marrow, may be necessary to achieve therapeutically effective amounts.

The invention is useful for enchancing the success of cell transplantation procedures and, preferably, transplantation of stem cells, progenitor cells and red blood cells such as, for example, in red blood cell engraftment processes. Stern cells and other types of cells for transplantation may be obtained from bone marrow, cord blood, leukophoresis procedures, or peripheral blood collection. In blood collection, cells are obtained from, for example, adult patients and cultured *in vitro* in the presence of cytokines such as Epo, growth factors *(e.g.* fibroblast growth factor, stem cell growth factor), bone morphogenic proteins, interleukin (IL) such as IL-1, iL-2, IL-3, Il-4, IL-5, IL-6, IL-7, *etc.,* and preferably IL-3, that stimulate proliferation and/or differentiation of the cells. The patient is than subjected to therapy such as chemotherapy or radiotherapy that destroys one or more cell populations in the body. Cultured cells are than mobilized back into the patient to re-populate the one or more of the now cell depleted systems including organ systems. These methods can increase the success of red blood cell engraftment processes and other transplantation procedures. Methods are also useful when the patient is immunosuppressed such as in association with an infection, or by design subsequent to organ transplantation. Infections that can cause immunosuppression include viral infection such as, for example, infection of Epstein Barr virus, adenovirus, cytomegalovirus and other herpes viruses, and retroviruses including T cell and B cell viruses which can induce disorders associated with acquired immunodeficiency syndrome.

The invention is additionally useful for a method for hemodilution comprising administering a composition of the invention to a patient undergoing a hemodilution process. Hemodilution involves the extraction or removal of blood for a patient prior to a treatment therapy. Treatment may require subsequent re-population with the patient's blood cells or infusion of blood during treatment such as during surgery. Compositions of the invention can be administered before blood removal to maximize the hemoglobin or erythroid cell content of the blood, or after removal to maximize recovery of the patient hemoglobin or erythroid cell levels, or both before and after therapy.

### The Detection of Erythroid Progenitors in the Colony Formation Assay

Hemoglobin enhances the growth of erythroid progenitors, notably the BFU-E progenitor population as shown by a well-known technique in the field of erythropoiesis research. This technique, referred to as the colony formation assay (CFA), is the most widely used biological assay to identify and enumerate erythroid progenitors present in hematopoietic tissue such as blood and bone marrow. Cell populations containing erythroid progenitors are plated in semi-solid suspensions of methyl cellulose, agar, low melting agarose or related substances in nutrient culture medium containing 1 - 3 U/ml Epo and 10 ng/ml IL-3 to specifically stimulate erythroid progenitors. Plates are incubated at 37°C for 14 days during which time the erythroid progenitors form characteristic hemoglobinized (red) colonies. Two distinct, but related erythroid progenitors can be distinguished based on colony size and morphology: BFU-E, the most primitive recognizable erythroid progenitor forms large multilobular colonies whereas the more mature CFU-E forms smaller spherical colonies. Under these conditions other myeloid progenitors, notably the colony forming unit - granulocyte-macrophage (CFU-GM), also form morphologically identifiable nonhemoglobinized (white) colonies which are readily distinguished from erythroid colonies. Additional cytokines, notably IL-1, IL-6, stem cell factor, fit-3 ligand, and granulocyte-macrophage colony stimulating factor, may be included in the CFA to stimulate both erythroid and nonerythroid progenitor proliferation.

### Hematopoietic Progenitors in Human Umbilical Cord and Adult Peripheral Blood

Umbilical cord blood is a rich source of hematopoietic progenitors. For this reason, umbilical cord blood is often used as a bone marrow replacement The approximate progenitor numbers in cord blood and adult blood are shown Table 1. In adult peripheral blood, hematopoietic progenitors are present at reduced levels compared to cord blood.

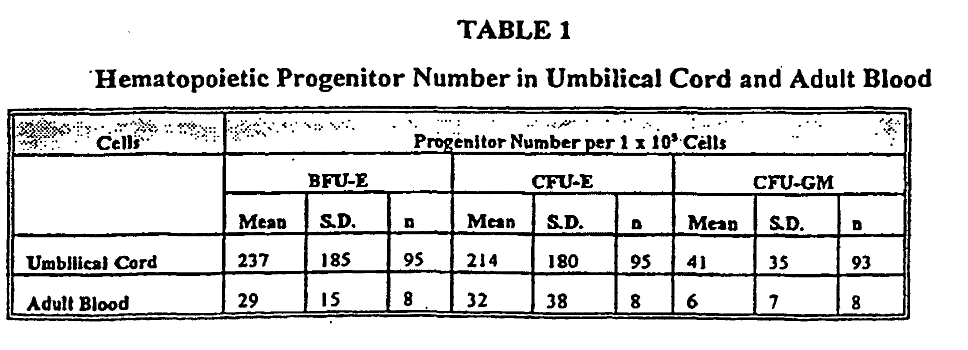

### Hemoglobin Stimulates Erythroid Progenitor Differentiation

The ability of purified adult hemoglobin to stimulate erythroid progenitor differentiation has also been studied using a liquid culture system containing Epo. In this liquid culture system hemin is known to accelerate erythroid differentiation of progenitors obtained from normal adult peripheral blood, and to selectively increase fetal hemoglobin production in these cells. Primary low density mononuclear cells (LDMNC) isolated from cord blood were tested in this culture system. Erythropoietin (2 U/ml) was found to stimulate erythroid differentiation of cord blood LDMNC in liquid culture resulting in an increase in glycophorin A and CD71 co-expression as well as benzidine staining indicative of hemoglobin production. The addition of HAo (7.8 - 15.6 µM) to the cultures results in a further increase in glycophorin A/CD71 co-expression (80% vs. 53% in the Epo only control) and benzidine staining (82% vs. 43% in the Epo only control) after 20 days in culture. These data are consistent with a role for hemoglobin in the stimulation of erythroid differentiation in addition to the stimulation of erythroid progenitor proliferation.

### Hemoglobin Stimulates Erythroid Progenitors at Low Concentrations of Erythropoietin

A particularly preferred embodiment of the present invention is the stimulation of erythroid progenitors by hemoglobin in the presence of reduced concentrations of erythropoietin. The Epo used in the experimental work reported herein is recombinant human Epo (tissue culture grade) obtained from R&D Systems (catalog number 286-EP), although any biologically active form of Epo should work equally well. Quantities are expressed in international activity units (U) per ml of solution. It is used in the form of a sterile-filtered solution in 50% PBS with carrier. Normally 2 U/ml Epo is used to stimulate maximal progenitor growth in the CFA. To determine the erythropoietic activity of hemoglobin under sub maximal Epo stimulation, the effects of HAo (15.6 *µ*M), HEMOLINK^{TM} (15.6 *µ*M), hemin (100 *µ*M) and FeCl₃ (250 *µ*M) were tested in the presence of 0.2 and 0 U/ml Epo at both ambient and 5% O₂. Few colonies form on the control plates (vehicle only) containing only 0.2 U/ml Epo. The pale pink color of these colonies indicates that they are poorly hemoglobinized. Surprisingly, hemin, HAo and HEMOLINK™, but not FeCl₃, all increase the number of BFU-E colonies in the presence of only 0.2 U/ml Epo at ambient oxygen tensions. Similar results are observed at 5% O₂ under which conditions more erythroid progenitor colonies are produced than at ambient oxygen tensions. The dark red color of colonies which form in the presence of HAo, HEMOLINK^{TM} or hemin at 0.2 U/ml Epo indicates that they are much more highly hemoglobinized than the control colonies. These data show that hemoglobin can promote Epo- induced erythroid progenitor proliferation (increased colony number) and differentiation (increased hemoglobinization of the colonies). No erythroid colonies were observed under any conditions in the absence of Epo. The reported role of Epo in inducing only differentiation and prolonging the G1 or quiescent phase of the erythroid cell cycle at low doses suggests that hemoglobin is capable of serving as a mitogen and providing a necessary and complementary proliferative signal that is otherwise only provided by high doses of Epo. Thus, Epo is essential for erythroid progenitor growth but hemoglobin can considerably lower the amount of Epo required to achieve an effective erythropoietic response. This discovery has important therapeutic applications for the use of hemoglobin in the treatment of anemias due to decreased Epo production, either alone or in conjunction with conventional Epo therapy.

This invention demonstrates that hemoglobin can partially substitute for Epo in the stimulation of erythropoiesis. Using the colony formation assay, comparable levels of erythroid progenitors proliferated in one-tenth the amount of Epo typically used (0.2 U/ml vs. 2 U/ml) when 1.0 mg/ml of purified hemoglobin, native or crosslinked, was added to the culture. Thus, any anemia which can be treated by Epo therapy could theoretically be managed by the combination therapy of reduced Epo doses supplemented with hemoglobin administration. Indications which are currently being treated with exogenous Epo, and thus those which are amenable to Epo/hemoglobin combination therapy, include end stage renal disease (including chronic renal failure), anemias associated with rheumatoid arthritis, cisplatin-associated anemia, solid tumors, lymphomas treated with or without chemotherapy, multiple myeloma, AIDS or myelodysplastic disorders. Epo therapy has also been used in patients undergoing allogeneic bone marrow transplants (Sowade, Blood 89:411-18, 1997) and infants suffering from late anemia associated with Rhesus hemolytic disease (Zachee, Drugs 49:536-47, 1995). Recently, Epo therapy has been granted U. S. approval for use in anemic patients scheduled to undergo elective, non-cardiac, non-vascular surgery to reduce the need for allogeneic blood transfusions (SCRIP, 2194:19, 1997). Other conditions which are associated with low endogenous Epo levels but are not currently being treated with exogenous Epo include malaria-induced anemia (Burgmann, Am. J. Trap. Med. Hyg. 54:280-83, 1996) and premature neonates (Zachee, Drugs 49:536-47, 1995). These conditions can also benefit from combination Epolhemoglobin therapy.

### Hemoglobin Reduces the Amount of Epo Required to Treat of Anemia

The current regiment for the treatment of anemia with exogenous Epo is to achieve a hematocrit between 30 - 36% (Dunn and Markham, Drugs 51:299-318, 1996). Presently, the safety and added benefits of maintaining the hematocrit between 39 - 45% (corresponding to normal levels) is being assessed for Epo therapy. The amount of Epo administered for the treatment of anemia is dependent on the type of anemia, the route of Epo administration and the level of endogenous Epo. Epo doses of 225 U/kg/week administered three times weekly or 429 U/kg/week administered once a week have been reported to maintain a hematocrit of 33 - 40% in patients with end-stage renal disease. Assuming an 85 ml blood volume/kg these doses range from 0.9 - 5.1 U/ml in the blood volume. Based on the demonstration that *in vitro* hemoglobin (1.0 mg/ml) can compensate for a reduction in Epo dose from 2 to 0.2 U/ml, then Epo administration could be reduced to 0.27 - 0.51 U/ml (equivalent to 22.5 to 42.9 U/kg/week) if supplemented with 1.0 mg/ml hemoglobin (equivalent to 85 mg/kg/week). Examples of other disorders currently being treated with exogenous Epo and typical Epo doses which are administered to these patients are listed in Table 2.

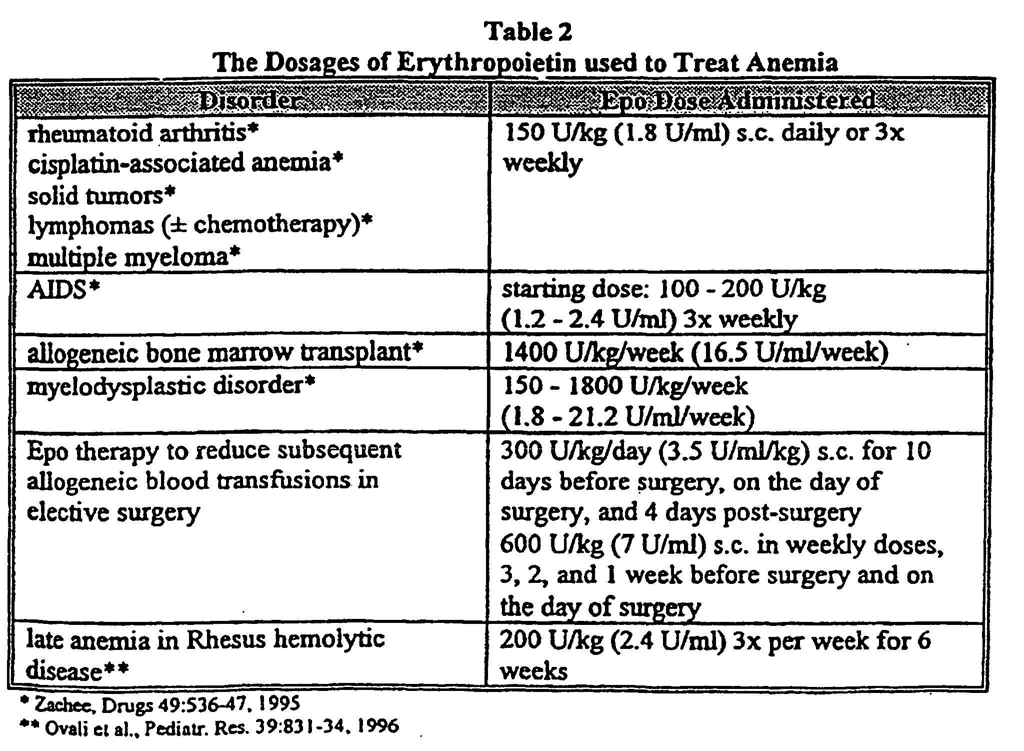

The doses of Epo used for the treatment of the disorders in Table 2 are comparable to those used for the treatment of chronic renal failure and thus these respective anemias should be amenable to Epo/hemoglobin combination therapy.

Another strategy for the co-administration of Epo and hemoglobin is to reduce the number of Epo administrations required during therapy. Several Epo therapies follow the regimen of three weekly injections, either intravenously or subcutaneously. Multiple low dose or single high dose injections of Epo are required due to its relatively short half-life *in vivo.* The half-life of Epo in plasma has been estimated to be 5.6 - 8.8 hours after an intravenous injection and 11.2 - 21.1 hours after a subcutaneous injection, however only 21.5 - 46.6% of the Epo administered subcutaneously is actually absorbed into the bloodstream (Dunn and Markham, Drugs 51:299-318, 1996). HEMOLINK^{TM} has a longer half-life. At therapeutic doses of 0.4 g HEMOLINK™/kg body weight the half-life of the 64 kD fraction, which represents ~33% of the crosslinked hemoglobin species, is ~7 hours whereas that of the >64 kD fraction, representing ~66% of the crosslinked hemoglobin species, is ~21 hours (Table 3). Longer half-life of the cross-linked hemoglobin can therefore compensate for Epo which may decline at a much faster rate. Thus, hemoglobin co-administered with Epo could prolong the effectiveness of Epo and therefore this combination therapy can be used to reduce the required frequency of Epo administrations.

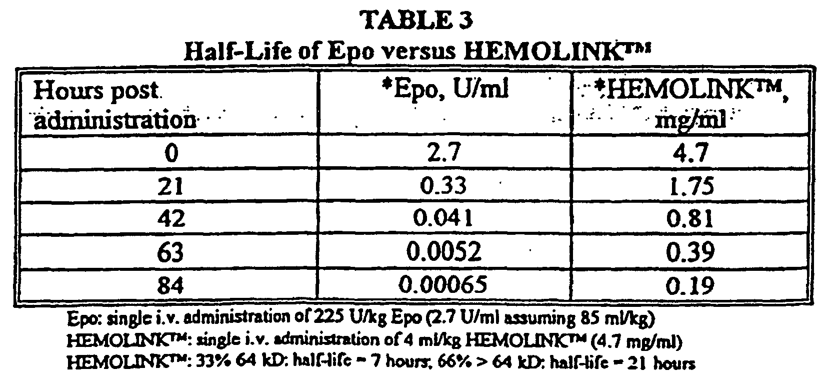

The following examples illustrate embodiments of the invention, but should not be viewed as limiting the scope of the invention.

### Examples

### Example 1 Purified Adult Hemoglobin, HEMOLINK^{TM}. Hemin and FeCl₃ Support the Expansion of Erythroid Progenitors from Umbilical Cord Blood

Adult hemoglobin was purified according to U.S. Patents 5,439,591 and 5,545,328 to obtain HAo and HEMOLINK™ was prepared from HAo according to U.S. Patent 5,532,352. Human umbilical cord blood intended for discard was collected from the umbilical vein of placentas immediately postpartum into 10 ml vacutainer tubes containing 143 USP units/ml sodium heparin. Low density mononuclear cells were separated from red blood cells by centrifugation over a 1.077 g/ml density gradient. Cells removed from the plasma/density gradient interface were incubated overnight in a tissue culture flask with 10 ml cell culture medium containing 10% fetal bovine serum, and non-adherent LDMNC were further purified with an additional density gradient step. The LDMNC were then plated into colony formation assays (CFA) on the second day of isolation. In the CFA, LDMNC were plated at a density of 1 x 10⁵ cells/ml in Iscoves modified Dulbeccos cell culture medium containing 0.8% methyl-cellulose, 30% fetal bovine serum, 1% bovine serum albumin, 0.1 mM 2-mercaptoethanol and 2 mM L-glutamine. Unless indicated otherwise, under standard CFA conditions 2 U/ml Epo and 10 ng/ml IL-3 are added to the formulation to stimulate hematopoietic progenitor growth, specifically BFU-E, CFU-E and CFU-GM.

HAo, HEMOLINK^{TM}, hemin and FeCl₃ were added to the cultures at the concentrations indicated in Table 4. Each condition was tested in duplicate in from 2 - 18 independent experiments. Cultures were maintained in a humidified incubator at 37°C, 5% CO₂ and ambient oxygen tensions. The number of hematopoietic progenitors was scored between days 13 - 15 by counting the number and types of colonies present.

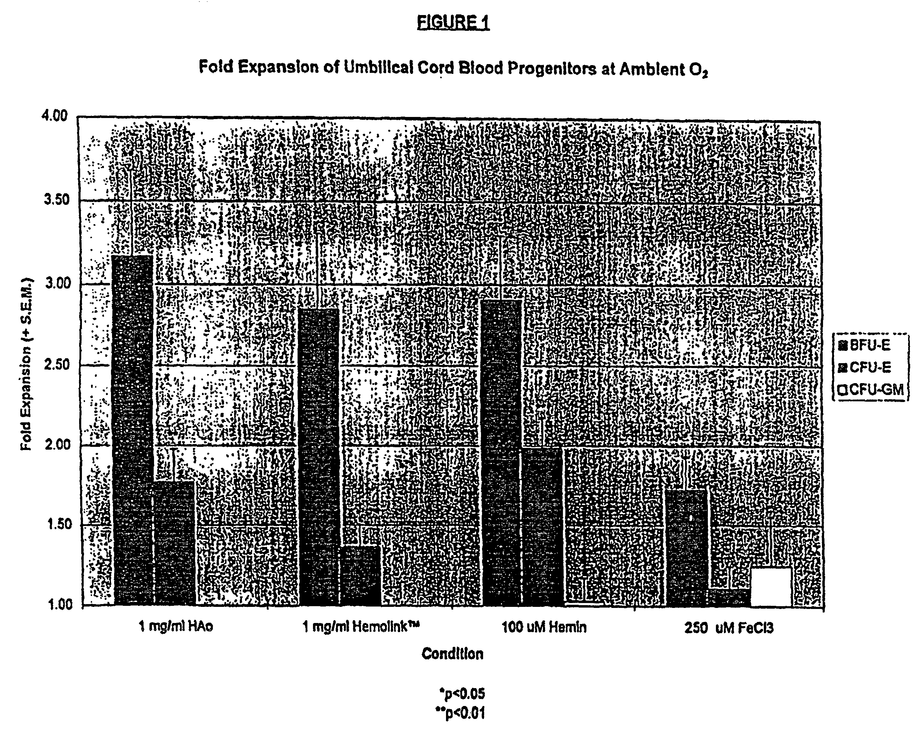

The mean fold increase in cord blood BFU-E, CFU-E, and CFU-GM progenitor number produced by 1.0 mg/ml HAo (15.6 *µ*M; n *=* 18) or HEMOLINK^{TM} (15.6 *µ*M; n = 6), 100 *µ*M hemin (n = 17 - 18) or 250 *µ*M FeCl₃ (n = 8) is shown in Figure 1. As indicated, 1.0 mg/ml HAo stimulated a 3.2-fold (p < 0.01) increase in BFU-E and a 1.8-fold increase in CFU-E (p < 0.01). The fold increase in colonies is expressed relative to control cells treated only with the respective diluents (vehicles) used in the preparation of the test compounds (fold expansion = 1.0; *p < 0.05, ** p < 0.01). The fold increase in progenitor number was dose-dependent over the range 10 - 1000 *µ*g/ml HAo (Table 4) reaching maximum erythroid progenitor stimulation at 1.0 mg/ml HAo (15.6 *µ*M). HEMOLINK^{TM}, at 1.0 mg/ml (15.6 *µ*M) produced a similar increase in progenitor number to 1.0 mg/ml HAo. Hemin and FeCl₃ also showed a dose-dependent increase in the stimulation of erythroid progenitors, reaching their respective maxima at 100 *µ*M. Although HAo, HEMOLINK^{TM} and hemin, but not FeCl₃, stimulated a significant increase in CFU-E, the overall magnitude of CFU-E stimulation was much less than obtained for BFU-E. CFU-GM numbers were unaffected by FeCl₃ and hemin- The apparent decrease in CFU-GM treated with HAo and HEMOLINK™ results from an under-estimation of CFU-GM numbers due to the formation of white granular hemoglobin precipitates in the CFA which render nonhemoglobinized colonies difficult to score. As indicated in Table 4, CFU-GM numbers were only decreased at the highest HAo or HEMOLINK™ concentrations where hemoglobin precipitation was greatest.

**TABLE 4**

| **Effect of FeCl**_{**3**}**, Hemin. HAo and HEMOLINK™ on Progenitor Number** | | | | | | | |
|---|---|---|---|---|---|---|---|
| | | **Progenitor Type** | | | | | |
| Agent | Dose | BFU-E | n | CFU-E | n | CFU-GM | n |
| FeCl₃, *µ*M | 1.0 | 1.2 | 2 | 1.0 | 2 | 1.2 | 2 |
| | 10 | 1.4 | 2 | 1.2 | 2 | 1.0 | 2 |
| | 100 | *2.1 | 5 | 1.2 | 5 | 0.8 | 5 |
| | 250 | *1.7 | 8 | 1.1 | 8 | 1.3 | 8 |
| Hemin, *µ*M | 1.0 | 0.9 | 5 | 1.2 | 5 | 1.5 | 4 |
| | 10 | 1.5 | 5 | *1.6 | 5 | 1.4 | 4 |
| | 64 | *2.3 | 6 | 1.8 | 6 | 1.2 | 5 |
| | 100 | **2.9 | 18 | **2.0 | 18 | 1.0 | 17 |
| | 250 | 2.2 | 4 | 2.2 | 4 | 1.4 | 4 |
| HAo, *µ*g/ml | 10 | 1.1 | 5 | 1.2 | 5 | 1.4 | 4 |
| | 100 | 1.3 | 5 | 1.5 | 5 | 1.5 | 4 |
| | 500 | **1.7 | 11 | *1.4 | 11 | 1.2 | 9 |
| | 1000 | **3.2 | 18 | **18 | 18 | ** 0.5 | 18 |
| HEMOLNK™, *µ*g/ml | 1000 | *2.8 | 6 | *1.4 | 6 | * 0.6 | 6 |

| | | | | | | | |
|---|---|---|---|---|---|---|---|
| *p<0.05, | | | | | | | |
| **p<0.01 | | | | | | | |

### Example 2 Enhanced Growth of Cord Blood Erythroid Progenitors Stimulated by HAo, HEMOLINK^{TM} and Hemin is further Enhanced at Low Oxygen Tension

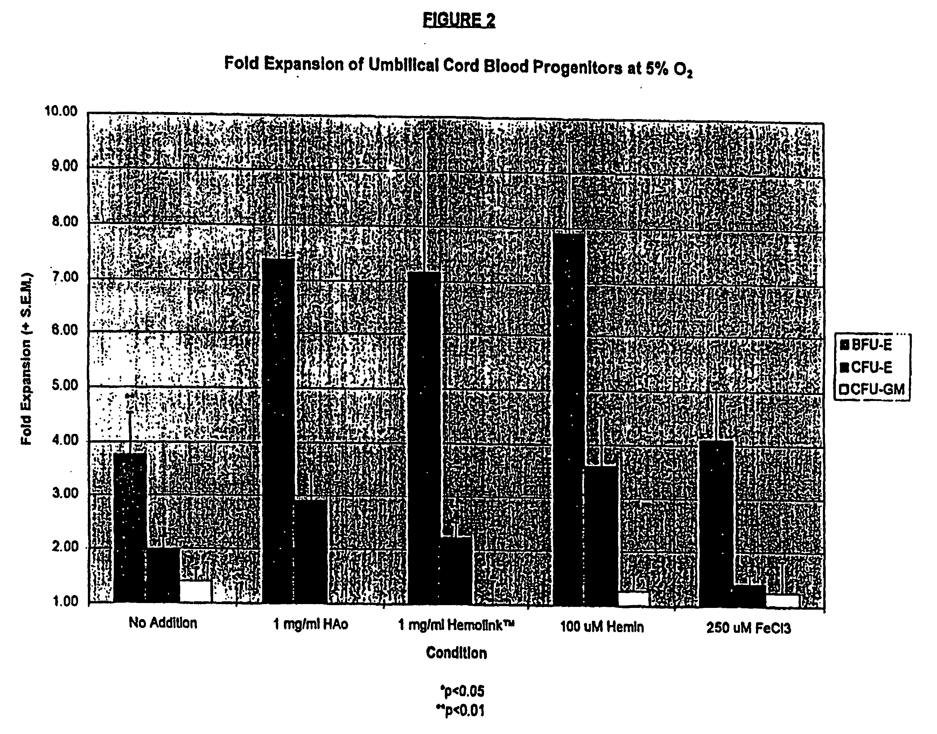

Erythroid progenitor colony formation assays were conducted as described in Example 1 except that cultures were incubated at either ambient oxygen tensions (~20% O₂) or 5% O₂. The reduced oxygen tension of 5% O₂ approximates the physiological intravascular oxygen tension in mammals. The mean fold increase in cord blood BFU-E, CFU-E and CFU-GM progenitor number at 5% O₂ produced by 1.0 mg/ml HAo (15.6 *µ*M; n = 11) or HEMOLINK™ (15.6 *µ*M; n = 6), 100 *µ*M hemin (n = 10), 250 *µ*M FeCl₃ (n = 6) or no addition (n = 17 - 18), is shown Figure 2. Results are expressed relative to control cells treated only with the respective vehicles and cultured at ambient oxygen tensions (~20% O₂; fold expansion = 1.0; *p < 0.05, ** p < 0.01).

More erythroid progenitor colonies formed in the untreated controls at 5% O₂ than at ambient oxygen tensions (3.8-fold and 2-fold increase in BFU-E and CFU-E, respectively). The oxygen-dependent increase in erythroid progenitors was further enhanced in the presence of HAo, HEMOLINK™ or hemin. Again these three agents stimulated comparable increases in BFU-E and CFU-E (~8-fold and ~3-fold, respectively) relative to the untreated controls at ambient oxygen tensions. FeCl₃ did not increase the fold expansion of erythroid progenitors beyond that of the untreated controls at 5% O₂ (no addition):

### Example 3 HAo and HEMOLINK^{TM} Promote Cord Blood Erythroid Progenitor Proliferation under Reduced EPO Concentrations

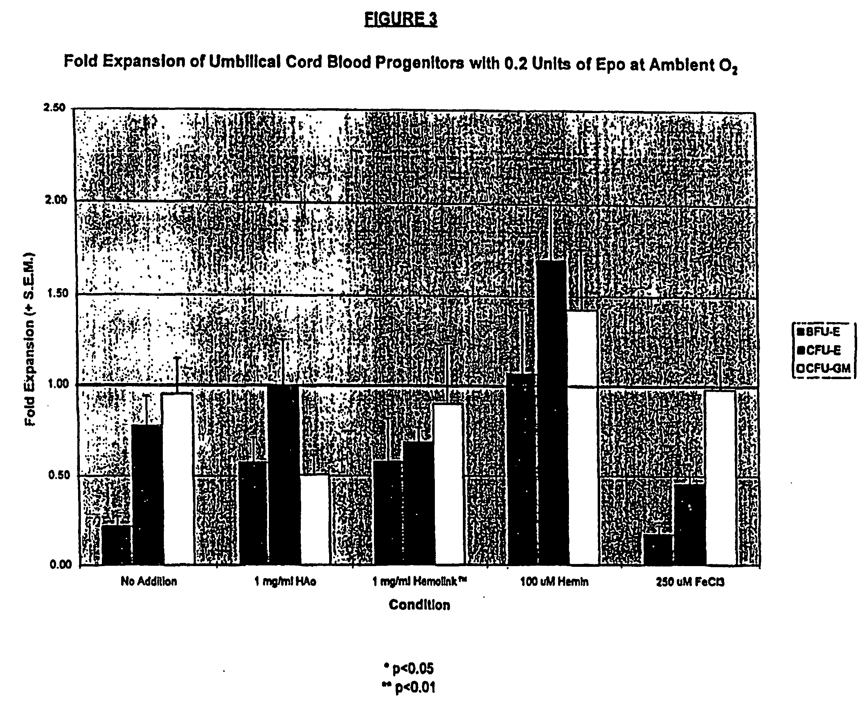

Erythroid progenitor colony formation assays were conducted as described in Example I except that cultures were initiated in the presence of Epo concentrations ranging from 0 - 2 U/ml. The mean fold increase in cord blood BFU-E, CFU-E and CFU-GM progenitor number produced by 1.0 rng/ml HAo (15.6 µM; n = 9) or HEMOLINK™ (15.6 µM; n = 6), 100 µM hemin (n = 8), 250 µM FeCl₃ (n = 6), or no addition (n = 9 - 10) in the presence of reduced Epo concentrations (0.2 U/ml) is shown in Figure 3. Cultures were maintained at ambient oxygen tensions (~20% O₂) for 14 days. Results are expressed relative to control cells at 2 U/ml Epo treated only with the respective vehicles (solid line at fold expansion = 1.0; *p < 0.05, ** p < 0 01). Erythroid progenitor colonies were undetectable in the absence of Epo.

Reducing the Epo concentration 10-fold from the standard concentration of 2 U/ml to 0.2 U/ml resulted in a 5-fold decrease in BFU-E progenitors. The addition of hemin to such plates, generated the same number of BFU-E as the untreated controls at 2 U/ml Epo, and HAo and HEMOLINK™, but not FeCl₃, stimulated a 3-fold increase in BFU-E number over the untreated controls (no addition) at 0.2 U/ml Epo. Hemin, HAo or HEMOLINK™ could not support the growth of erythroid progenitor colonies in the absence of Epo, indicating the specificity of these agents for the erythroid lineage.

### Example 4 HAo and HEMOLINK^{TM} Promote Cord Blood Erythroid Progenitor Proliferation in the Presence of Reduced EPO Concentrations at Low Oxygen

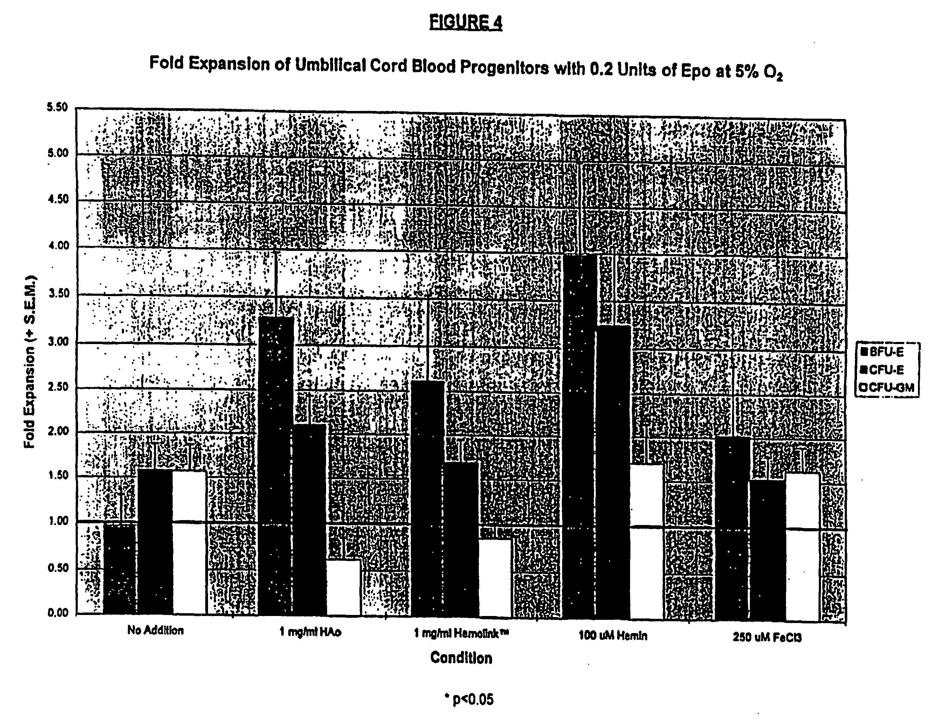

Erythroid progenitor colony formation assays were conducted as described in Example 3 except that cultures were incubated at 5% O₂. The mean fold increase in cord blood BFU-E, CFU-E and CFU-GM progenitor number at 5% O₂ produced by 1.0 mg/ml HAo (15.6 *µ*M; n = 9) or HEMOLINK™ ( 15.6 *µ*M; n=6), 100 *µ*M hemin (n = 8) or 250 *µ*M FeCl₃ (n=6) or no addition (n = 9 - 10) in the presence of reduced Epo concentrations (0.₂ U/ml) is shown in Figure 4. Cultures were maintained at 5% O₂ for 14 days. Results are expressed relative to control cells at 2 U/ml Epo treated only with the respective vehicles and cultured at ambient oxygen tensions (~20% O₂) for 14 days (solid line at fold expansion = 1.0; *p < 0.05). At 5% O₂, the number of erythroid progenitors was maintained in CFAs containing a 10-fold lower concentration of Epo (0.2 U/ml) than the standard dose of 2 U/ml. Under these conditions, 100 *µ*M hemin stimulated a 4-fold and 3.2-fold increase in BFU-E and CFU-E colonies, respectively, as compared to the control plates at 2 U/ml Epo at ambient oxygen tensions. Similarly, 1.0 mg/ml HAo (15.6 *µ*M) and HEMOLINK™ (15.6 *µ*M) resulted in 3.3 and 2.6-fold increases in BFU-E, and 2.1 and 1.7-fold increases in CFU-E, respectively, over the controls. However, 250 *µ*M FeCl₃ did not significantly increase the number of erythroid colonies stimulated under these conditions, similar to the results obtained with FeCl₃ at ambient oxygen tensions (Figure 3).

### Example 5 Erythroid Colonies which form in the Presence of HAo and HEMOLINK^{TM} are Larger and Produce more Hemoglobin per Cell

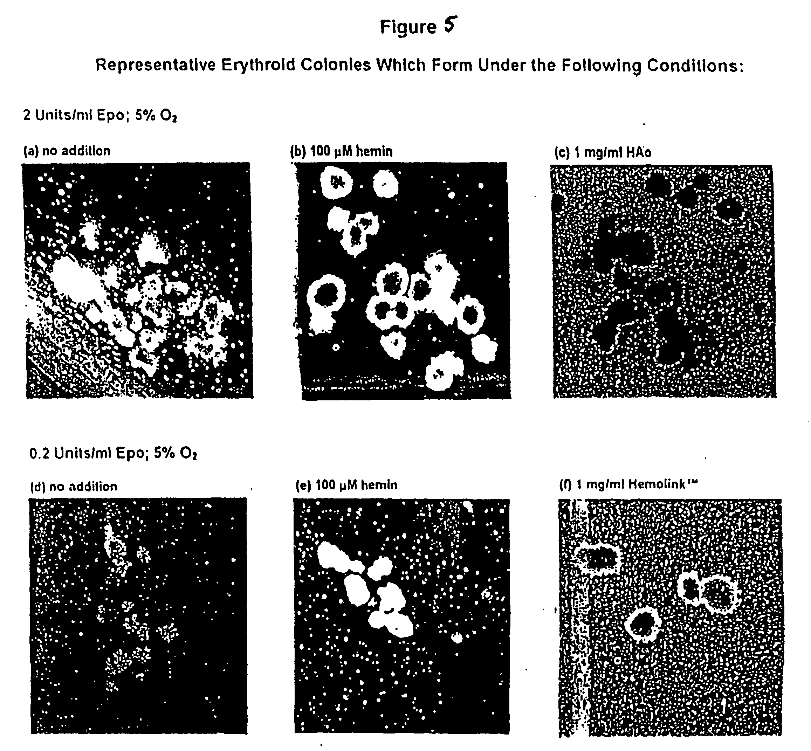

Shown in Figure 5 are photographs of representative cord blood BFU-E colonies which form in CFA at day 14 in the presence of 2 U/ml Epo at 5% O₂ under the following conditions: (a) no addition (vehicle only), (b) 100 *µ*M hemin and (c) 1.0 mg/ml (15.6 *µ*M) HAo. Photographs also include representative colonies that formed in the presence of 0.2 U/ml Epo and 5% O₂, under the following conditions: (d) no addition (vehicle only), (e) 100 *µ*M hemin and (f) 1.0 mg/ml (15.6 *µ*M) HEMOLINK^{TM}. Erythroid colonies which formed in the presence of HAo or HEMOLINK^{TM} were larger and redder than colonies on the untreated control plates (vehicle only) or those to which hemin was added. These observations are consistent at both 2 and 0.2 U/ml Epo and at either ambient or 5% O₂. The colonies which formed in the presence of hemin (100 *µ*M) under similar Epo concentrations and oxygen tensions were mostly smaller and less red in color than HAo- or HEMOLINK™-treated cells. The pale pink erythroid colonies present at 0.2 U/ml Epo in the control plates indicates poor hemoglobin synthesis whereas the dark red colonies that formed in the presence of HAo or HEMOLINK^{TM} indicates the stimulation of significantly greater hemoglobin synthesis by exogenous hemoglobin.

Hemoglobin synthesis in erythroid colonies was analyzed by anion exchange high performance liquid chromatography (HPLC). Colonies were harvested from CFA plates and cell lysates were prepared after the progenitor colonies had been enumerated. Colonies were isolated from methyl cellulose by several washes in PBS. The cells were pelleted and lysed in 50 mM Tris, pH 8.8 and the cell debris removed by pelleting the cell lysate. The lysate supernatants were filtered through a 0.2 *µ*m filter prior to loading onto a POROS® HQ/H anion exchange HPLC column (PerSeptive Biosystems). The hemoglobin was eluted with an increasing NaCl gradient and the optical density (O.D.) monitored at a wavelength of 414 nm. The amount of hemoglobin present in the lysates was quantitated by comparison with standard hemoglobin solutions of adult (HbA) and fetal hemoglobin (HbF).

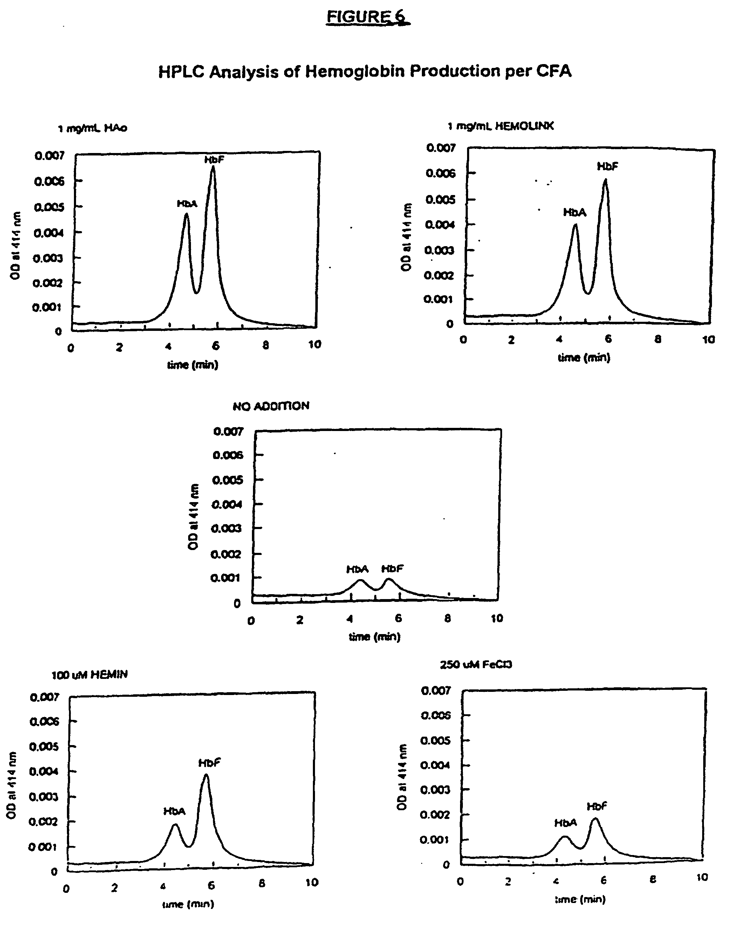

Representative HPLC profiles for hemoglobin produced by erythroid colonies that formed at 5% O₂ in the presence of 2 U/ml Epo with no addition (vehicle only), 100 *µ*M hemin, 1.0 mg/ml HAo (15.6 *µ*M) HAo, 1.0 mg/ml HEMOLINK^{TM} (15.6 *µ*M), or 250 *µ*M FeCl₃ are shown in Figure 6. The amount of hemoglobin produced under the above conditions is shown in Table 5.

**TABLE 5**

| **Effect of Hemin, HAo, HEMOLINK™ and FeCl**_{**3**} **on Hemoglobin Production** | | | |
|---|---|---|---|
| **Condition** | **adult Hb, *µ*g** | **fetal Hb, *µ*g** | **total Hb.*µ*g** |
| no addition | 3.7 | 4.4 | 8.1 |
| 100 *µ*M hemin | 8.7 | 19.4 | 28.1 |
| 1.0 mg/ml HAo | 22.6 | 33.1 | 55.7 |
| 1.0 mg/ml HEMOLINK™ | 20.5 | 28.0 | 48.5 |
| 250 *µ*M FeCl₃ | 5.0 | 9.5 | 14.5 |

Erythroid progenitors which formed in the presence of HAo or HEMOLINK™ contained the most hemoglobin. Both adult and fetal hemoglobin synthesis increased in response to HAo or HEMOLINK^{TM}. To exclude the possibility that the increase in adult hemoglobin was not simply due to contamination of the cell lysates with the exogenously added hemoglobin, HPLC analysis was conducted on cell lysates prepared from CFAs which contained G U/ml Epo, 1.0 mg/ml HAo or HEMOLINK™, and maintained at 5% O₂. Erythroid colonies did not form under these conditions. Hemoglobin was undetectable in these cell lysates suggesting that the added HAo or HEMOLINK^{TM} is adequately washed away, or removed from the cells and does not contaminate the cell lysates.

The amount of hemoglobin produced per erythroid cell was estimated by assuming 30,000 cells/BFU-E and 100 cells/CFU-E. Table 6 shows the amount of hemoglobin (pg) produced per cell from the above cultures.

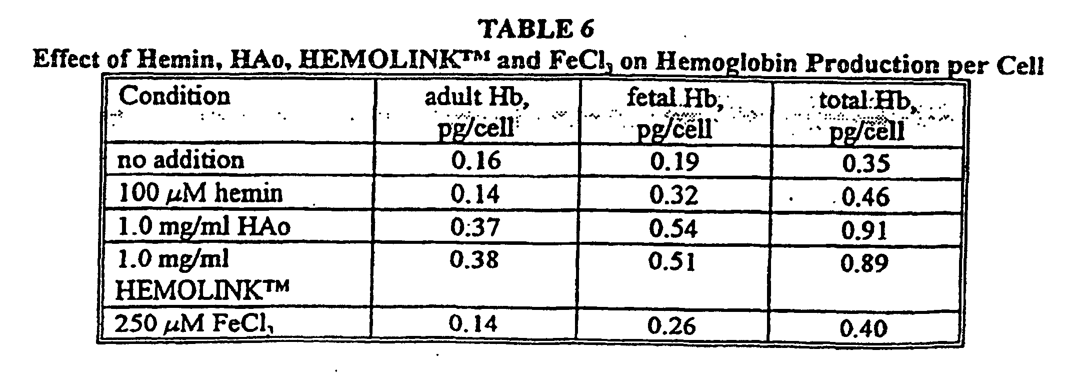

Thus, the increased redness of the erythroid colonies that formed in the presence of HAo and HEMOLINK^{TM}, as compared to those in the presence of hemin or FeCl₃, corresponds to an increase in hemoglobin production. These data suggest that exogenously added hemoglobin can directly stimulate the endogenous synthesis of both adult and fetal hemoglobin by erythroid cells.

### Example 6 HAo Enhances the Differentiation of Erythroid Cells in Liquid Culture

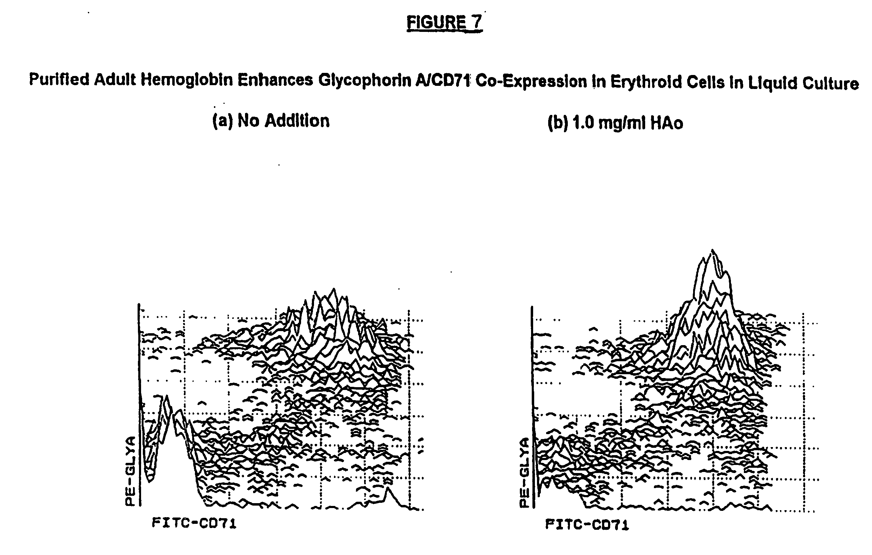

The effect of HAo on erythroid differentiation was tested on cord blood LDMNC. LDMNC were isolated as described in Example 1 and maintained in a liquid culture system that supports the expansion and differentiation of erythroid cells. 1.0 mg/ml HAo (15.6 *µ*M) was added to Epo-stimulated cultures and the cultured cells were analyzed by flow cytometry (Epics Elite, Coulter) after 20 days for the co-expression of the red cell-specific marker, glycophorin A, and the transferrin receptor, CD71. Co-expression of these two antigens is indicative of erythroid cell differentiation. Cultured cells were stained with anti-CD71 (abscissa) and anti-glycophorin A (ordinate) antibodies and the number of single-or double-stained cells (z-axis) analyzed. Cells simultaneously expressing both red blood cell membrane markers are located in the upper right region of each plot while those cells expressing neither antigen are located in the lower left region. The percentage of glycophorin A⁺/CD71⁺ cells was determined from analysis of at least 6,500 cells per sample. As demonstrated in Figure 7, HAo increased the percentage of cells co-expressing glycophorin A and CD71 compared to untreated cultures (80% vs. 53%, respectively), indicating that hemoglobin stimulates erythroid cell differentiation in addition to erythroid progenitor proliferation. These data also demonstrate that hemoglobin stimulates erythroid-specific gene expression and that exogenous hemoglobin is not simply used as a raw material in the production of new hemoglobin by differentiating erythroid progenitors.

### Example 7 Purified Adult Hemoglobin, HEMOLINK™, Hemin and FeCl₃ Support the Expansion of Erythroid Progenitors derived from Adult Peripheral Blood

Blood was collected with informed consent from the antecubital vein of healthy adult human volunteers into vacutainers containing 143 USP U/ml sodium heparin. Low density mononuclear cells (LDMNC) were isolated by centrifugation over a density gradient as described in Example 1. Plastic adherent cells were removed by an overnight incubation in a tissue culture flask and the non-adherent cells were placed directly in a CFA. Conditions for the CFA are similar to those described in Examples 1 - 4.

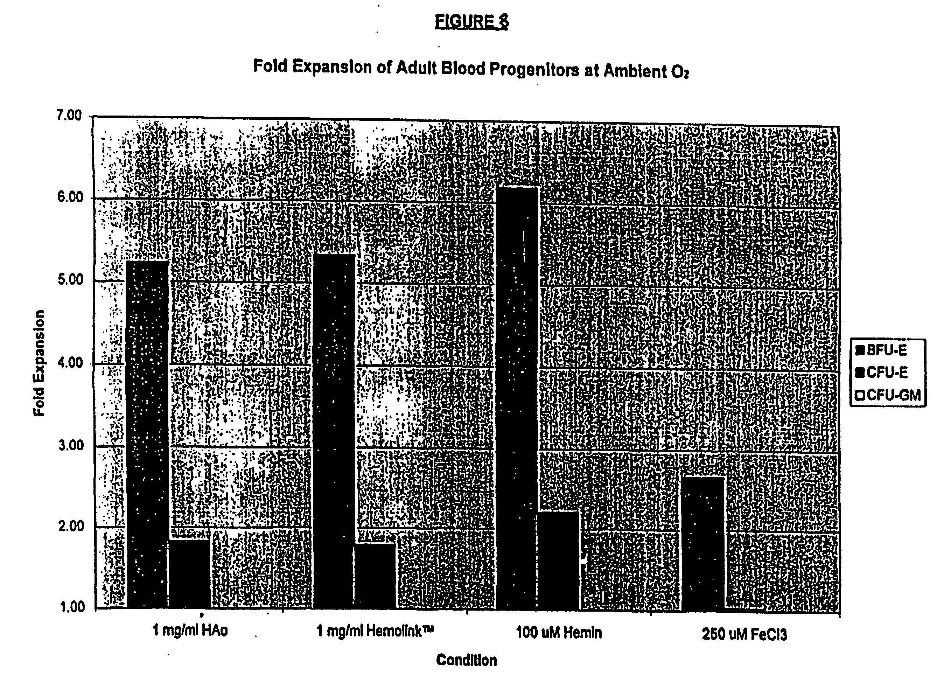

The mean fold increase in adult blood BFU-E, CFU-E and CFU-GM progenitor number produced by 1.0 mg/ml HAo (15.6 µM) or HEMOLINK^{TM} (15.6 µM), 100 *µ*M hemin or 250 µM FeCl₃ is shown in Figure 8 (n = 3). Cultures were maintained at ambient oxygen tensions (~20% O₂) for 14 days. The fold increase in colonies is expressed relative to control cells treated only with the respective vehicles. All agents evaluated stimulated an increase in BFU-E number at ambient oxygen tensions. The magnitude of this increase was greater for adult than cord blood LDMNC. HAo, HEMOLINK™ and hemin stimulated comparable increases (~5 - 6-fold) in BFU-E from adult blood LDMNC. FeCl₃ was less effective than HAo, HEMOLINK^{TM} or hemin, and stimulated a <3-fold increase in BFU-E number. HAo, HEMOLINK^{TM} and hemin, but not FeCl₃, also stimulated slight increases in the number of CFU-E progenitors.

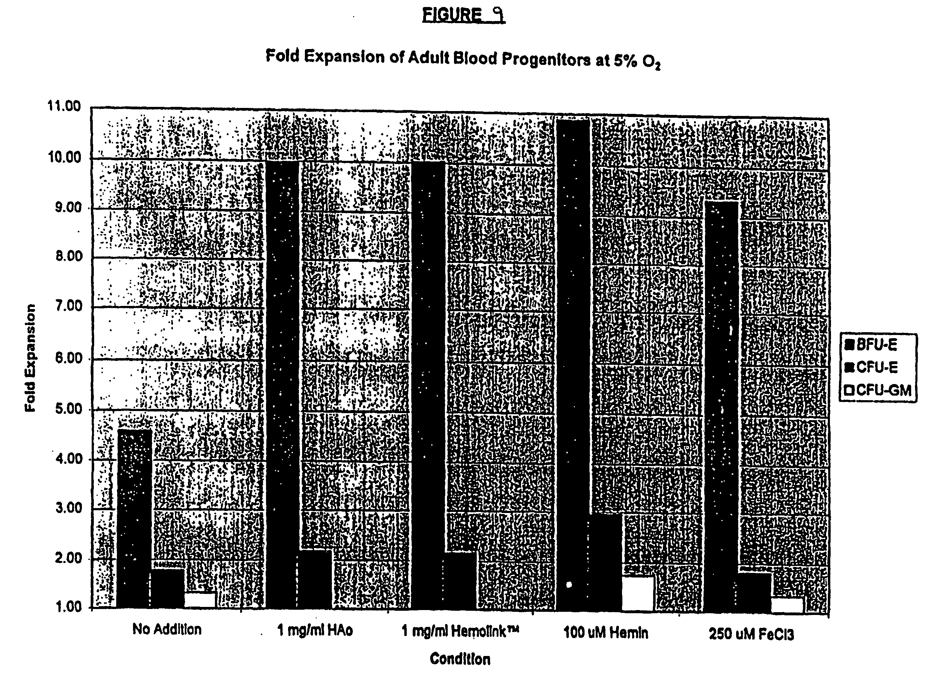

The mean fold increase in adult blood BFU-E, CFU-E and CFU-GM progenitor number at 5% O₂ is shown in Figure 9 with the same additives. Results are expressed relative to control cells treated only with the respective vehicles and cultured at ambient oxygen tensions (~20% O₂. Reduced oxygen tensions (5% O )₂enhanced the growth of BFU-E which was further increased by the addition of HAo, HEMOLINK^{TM}, hemin or FeCl₃. All agents stimulated a similar increase in BFU-E number (~9 - 11-fold) at 5% O₂ compared to untreated control cells at ambient oxygen tensions.

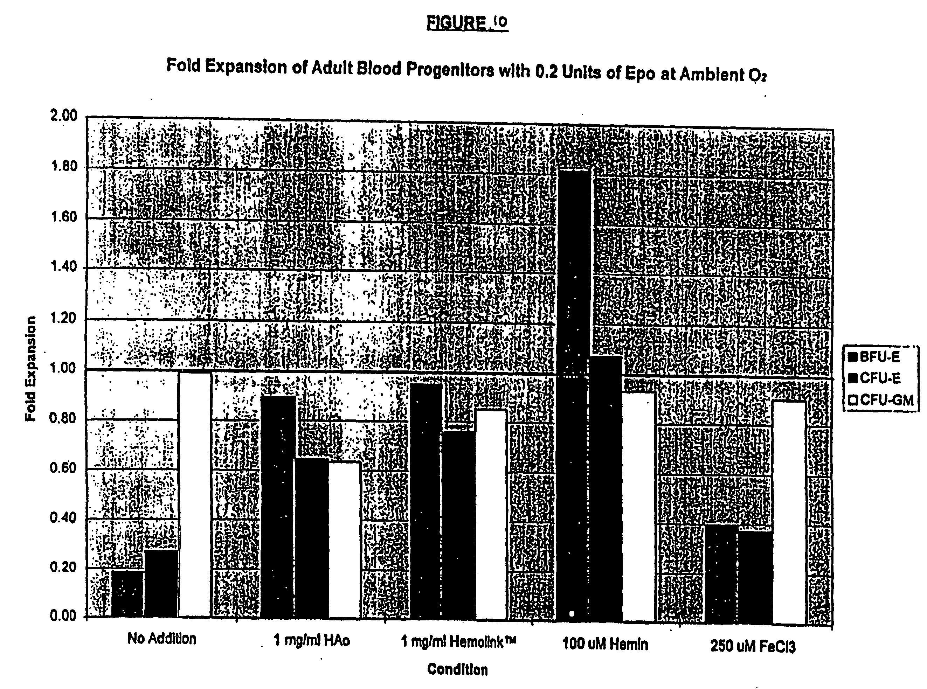

Mean fold increase in adult blood BFU-E, CFU-E and CFU-GM progenitor number in the presence of reduced Epo (0.2 U/ml) (n=3) at ~20% O₂ is shown in Figure 10 with and without (no addition) the additives used in Figure 8. Results are expressed relative to control cells at 2 U/ml Epo treated only with the respective vehicles (solid line at fold expansion = 1.0). Reducing the Epo concentration from the standard concentration of 2 U/ml to 0.2 U/ml resulted in a reduction in BFU-E and CFU-E number at ~20% O₂. As with cord blood, CFU-GM numbers were unaffected by reductions in Epo. The addition of HAo or HEMOLINK™ to CFAs containing 0.2 U/ml Epo stimulated ~4.5- and ~3.5-fold increases in BFU-E and CFU-E, respectively, compared to untreated controls (no addition), restoring the progenitor number to that obtained at 2 U/ml Epo. Hemin stimulated the greatest increase in BFU-E and CFU-E (~9- and ~6-fold, respectively) at 0.2 U/ml Epo versus the untreated controls (no addition). FeCl₃ stimulated only a ~2-fold increase in erythroid progenitor number at 0.2 U/ml Epo. HAo, HEMOLINK^{TM}, hemin and FeCl₃ could not support the growth of erythroid progenitors in the absence of Epo, indicating that Epo stimulation is essential for erythroid progenitor growth and differentiation.

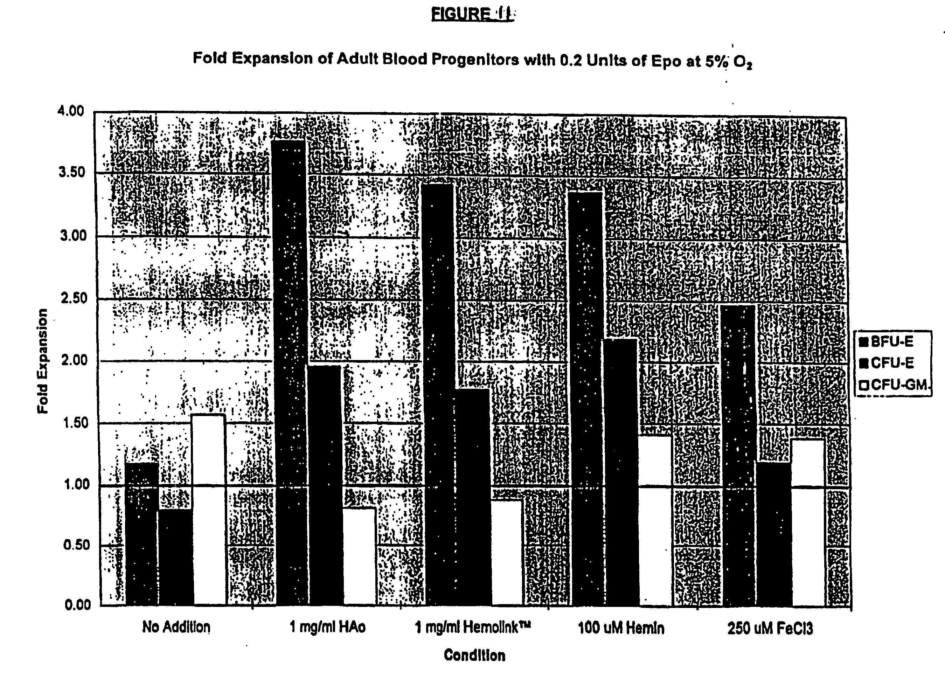

Mean fold increase in adult blood BFU-E, CFU-E and CFU-GM progenitor number at 5% O₂ in the presence of reduced Epo (0.2 U/ml) (n = 3) is shown in Figure 11 with and without the additives used in Figure 8. Results are expressed relative to control cells at 2 U/ml Epo treated only with the respective vehicles and cultured at ambient oxygen tensions (~20% O₂) for 14 days (solid line at fold expansion = 1.0). The number of erythroid progenitors stimulated at 5% O₂ and 0.2 U/ml Epo was similar to that obtained with 2.0 U/ml Epo maintained at ambient oxygen tensions (no addition). The addition of HAo, HEMOLINK™, hemin, and to a lesser extent FeCl₃, stimulated further increases in erythroid progenitor number, particularly BFU-E, over the untreated controls (no addition) cultured under the same conditions.

### Example 8 Purified Adult Hemoglobin, HEMOLINK^{TM}, Hemin and FeCl₃ support Expansion of Erythroid Progenitors from CD34⁺/CD38⁻ and CD34⁺/CD33⁻ Stem cells

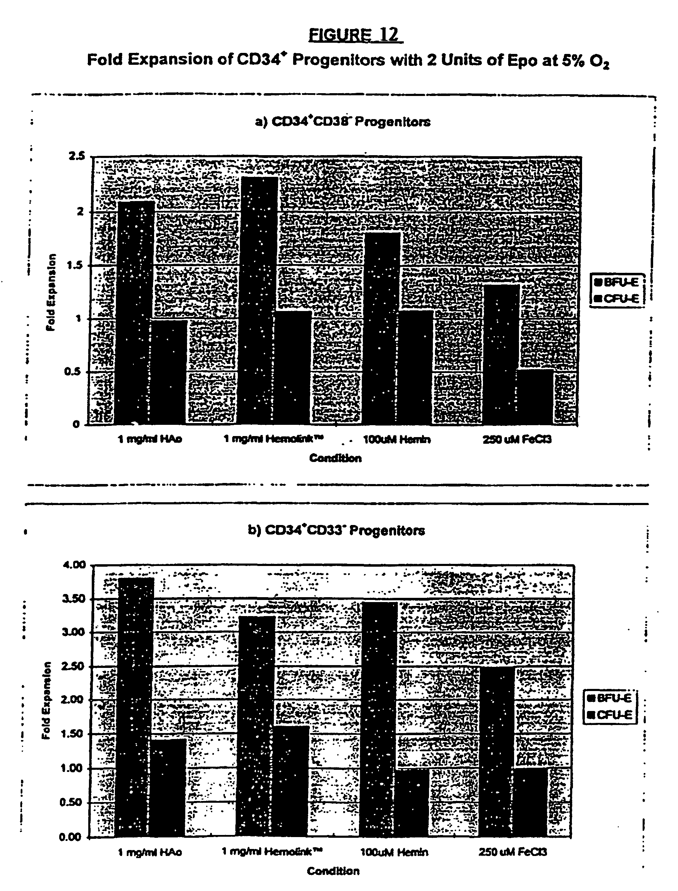

CD34⁺/CD38⁻ cells were isolated from cord blood LDMNC using the STEMSEP™ system (StemCell Technologies Inc., Vancouver, Canada). The CD34⁺/CD38⁻ cell purity was increased from <0.1% in unfractionated LDMNC to ~81% CD34⁺/CD38⁻ cells post fractionation as determined by flow cytometry. To obtain the CD34⁺/CD33⁻ cells, CD34⁺ cells were first isolated from cord blood LDMNC using the CEPRATE^{TM} LC system (CellPro Inc., Bothell, WA). The CD34⁺/CD33⁻ fraction was then enriched to ~94% purity by flow fluorescence activated cell sorting (FACS; Epics Elite, Coulter Electronics, Hialeah, FL). The colony formation assay was conducted as described in Examples 1 - 4, except that 1 x 10³ CD34⁺ cells/ml were plated versus 1 x 10⁵ unfractionated cord blood LDMNC/ml normally plated. Mean fold increase in BFU-E and CFU-E progenitor number produced from CD34⁺ CD38⁻ enriched (a; n = 2) and CD34⁺CD33⁻ enriched (b; n = 1 ) cord blood LDMNC stimulated by 1.0 mg/ml HAo (15.6 *µ*M) or HEMOLINK™ (15.6 *µ*M), 100 *µ*M hemin, 250 *µ*M FeCl₃ in the presence of 2 U/ml Epo is shown in Figure 12 Cultures were maintained at 5% O₂ for 14 days. The results are expressed relative to control cells treated only with the respective vehicles and cultured under the same conditions.

For both enriched progenitor cell populations, HAo, HEMOLINK^{TM}, hemin and to a lesser extent FeCl₃, stimulated an increase in BFU-E, as compared to untreated control cells cultured under the same conditions. The CFU-E numbers were not affected by the addition of any of the agents.

### Example 9 Statistics

Statistical comparisons in Figures 1 - 4 and Table 4 are based on the Student's t-test, using meaningfully paired observations and a 2-tail rejection region. The general null hypothesis for the comparisons in Figures 1 - 4 is H₀ there is no significant difference in fold expansion of progenitors with no addition, HAo, HEMOLINK™, hemin or FeCl₃ at ambient or 5% O₂ and 2.0 or 0.2 Units of Epo versus the fold expansion of progenitors with no addition at ambient O₂ and 2.0 Units of Epo. Significant differences are indicated by * (p < 0.05) and ** (p < 0.01).

## Claims

1. The use of erythropoietin in combination with hemoglobin for the manufacture of a medicament for enhancing erythropoiesis in a patient, wherein the amount of erythropoietin to be administered is from 22.5 to 42.9 U/kg/week and the amount of hemoglobin to be administered is 85 mg/kg/week.

2. The use according to claim 1, wherein enhancement of erythropoiesis in the patient is desirable due to anemia associated with chronic renal failure.

3. The use according to claim 1, wherein enhancement of erythropoiesis in the patient is desirable due to anemia associated with surgery.

4. The use according to claim 1, wherein enhancement of erythropoiesis in the patient is desirable due to anemia associated with chemotherapy.

5. The use according to claim 1, wherein enhancement of erythropoiesis in the patient is desirable due to drug-induced anemia.

6. The use according to claim 1, wherein enhancement of erythropoiesis in the patient is desirable due to anemia associated with rheumatoid arthritis.

7. The use according to claim 1, wherein enhancement of erythropoiesis in the patient is desirable due to anemia associated with AIDS.

8. The use according to claim 1, wherein enhancement of erythropoiesis in the patient is desirable due to anemia associated with allogenic bone marrow transplant.

9. The use according to claim 1, wherein enhancement of erythropoiesis in the patient is desirable due to anemia associated with myelodysplastic disorder.

10. The use according to claim 1, wherein enhancement of erythropoiesis in the patient is desirable due to anemia associated with Rhesus hemolytic disease.

11. The use according to any one of claims 1 to 10, wherein the hemoglobin is selected from natural hemoglobin, recombinant hemoglobin, cross-linked hemoglobin, oxyhemoglobin, deoxyhemoglobin, methemoglobin, NO-hemoglobin, CO-hemoglobin, or ferric hemoglobin.

12. The use according to claim 11, wherein the hemoglobin is cross-linked hemoglobin.

13. The use according to claim 12, wherein the hemoglobin is an O-raffinose cross-linked hemoglobin.

## Patentansprüche

1. Verwendung von Erythropoietin in Kombination mit Hämoglobin zur Herstellung eines Medikaments zur Steigerung der Erythropoiese bei einem Patienten, worin die zu verabreichende Erythropoietinmenge von 22,5 bis 42,9 U/kg/Woche liegt und die zu verabreichende Hämoglobinmenge bei 85 mg/kg/Woche liegt.

2. Verwendung nach Anspruch 1, worin eine Steigerung der Erythropoiese bei dem Patienten aufgrund einer mit chronischem Nierenversagen einhergehenden Anämie erwünscht ist.

3. Verwendung nach Anspruch 1, worin eine Steigerung der Erythropoiese bei dem Patienten aufgrund einer mit einem chirurgischen Eingriff einhergehenden Anämie erwünscht ist.

4. Verwendung nach Anspruch 1, worin eine Steigerung der Erythropoiese bei dem Patienten aufgrund einer mit Chemotherapie einhergehenden Anämie erwünscht ist.

5. Verwendung nach Anspruch 1, worin eine Steigerung der Erythropoiese bei dem Patienten aufgrund einer arzneimittelinduzierten Anämie erwünscht ist.

6. Verwendung nach Anspruch 1, worin eine Steigerung der Erythropoiese bei dem Patienten aufgrund einer mit rheumatoider Arthritis einhergehenden Anämie erwünscht ist.

7. Verwendung nach Anspruch 1, worin eine Steigerung der Erythropoiese bei dem Patienten aufgrund einer mit AIDS einhergehenden Anämie erwünscht ist.

8. Verwendung nach Anspruch 1, worin eine Steigerung der Erythropoiese bei dem Patienten aufgrund einer mit einer allogenen Knochenmarkstransplantation einhergehenden Anämie erwünscht ist.

9. Verwendung nach Anspruch 1, worin eine Steigerung der Erythropoiese bei dem Patienten aufgrund einer mit dem myelodysplastischen Syndrom einhergehenden Anämie erwünscht ist.

10. Verwendung nach Anspruch 1, worin eine Steigerung der Erythropoiese bei dem Patienten aufgrund einer mit durch Rhesus-Inkompatibilität bedingtem Morbus haemolyticus einhergehenden Anämie erwünscht ist.

11. Verwendung nach einem der Ansprüche 1 bis 10, worin das Hämoglobin aus natürlichem Hämoglobin, rekombinantem Hämoglobin, vernetztem Hämoglobin, Oxyhämoglobin, Desoxyhämoglobin, Methämoglobin, NO-Hämoglobin, CO-Hämoglobin oder Ferrihämoglobin ausgewählt ist.

12. Verwendung nach Anspruch 11, worin das Hämoglobin vernetztes Hämoglobin ist.

13. Verwendung nach Anspruch 12, worin das Hämoglobin ein O-Raffinosevemetztes Hämoglobin ist.

## Revendications

1. Utilisation d'érythropoïétine en combinaison avec de l'hémoglobine pour la fabrication d'un médicament pour améliorer l'érythropoïèse chez un patient, où la quantité d'érythropoïétine à administrer est de 22,5 à 42,9 U/kg/semaine et la quantité d'hémoglobine à administrer est de 85 mg/kg/semaine.

2. Utilisation selon la revendication 1, où l'amélioration de l'érythropoïèse chez le patient est désirable à cause d'une anémie associée à une insuffisance rénale chronique.

3. Utilisation selon la revendication 1, où l'amélioration de l'érythropoïèse chez le patient est désirable à cause d'une anémie associée à une intervention chirurgicale.

4. Utilisation selon la revendication 1, où l'amélioration de l'érythropoïèse chez le patient est désirable à cause d'une anémie associée à la chimiothérapie.

5. Utilisation selon la revendication 1, où l'amélioration de l'érythropoïèse chez le patient est désirable à cause d'une anémie induite par des médicaments.

6. Utilisation selon la revendication 1, où l'amélioration de l'érythropoïèse chez le patient est désirable à cause d'une anémie associée à une polyarthrite rhumatoïde.

7. Utilisation selon la revendication 1, où l'amélioration de l'érythropoïèse chez le patient est désirable à cause d'une anémie associée au SIDA.

8. Utilisation selon la revendication 1, où l'amélioration de l'érythropoïèse chez le patient est désirable à cause d'une anémie associée à une greffe de moelle osseuse allogénique.

9. Utilisation selon la revendication 1, où l'amélioration de l'érythropoïèse chez le patient est désirable à cause d'une anémie associée à un trouble myélodysplasique.

10. Utilisation selon la revendication 1, où l'amélioration de l'érythropoïèse chez le patient est désirable à cause d'une anémie associée à une maladie hémolytique Rhésus.

11. Utilisation selon l'une quelconque des revendications 1 à 10, où l'hémoglobine est sélectionnée parmi l'hémoglobine naturelle, l'hémoglobine recombinée, l'hémoglobine réticulée, l'oxyhémoglobine, la déoxyhémoglobine, la méthémoglobine, l'hémoglobine NO, l'hémoglobine CO ou l'hémoglobine ferrique.

12. Utilisation selon la revendication 11, où l'hémoglobine est de l'hémoglobine réticulée.

13. Utilisation selon la revendication 12, où l'hémoglobine est une hémoglobine réticulée O-raffinose.
